(19) Europäisches Patentamt
European Patent Office
Office européen des brevets

(11) Numéro de publication : **0 337 853 B1**

# (12) FASCICULE DE BREVET EUROPEEN

(45) Date de publication du fascicule du brevet : **20.05.92 Bulletin 92/21**

(51) Int. Cl.$^5$ : **A61K 7/13**

(21) Numéro de dépôt : **89400930.7**

(22) Date de dépôt : **05.04.89**

## (54) Procédé de conservation du pouvoir tinctorial du 5,6-dihydroxyindole et de ses dérivés en milieu aqueux, composition etprocédé de teinture.

(30) Priorité : **12.04.88 LU 87196**

(43) Date de publication de la demande : **18.10.89 Bulletin 89/42**

(45) Mention de la délivrance du brevet : **20.05.92 Bulletin 92/21**

(84) Etats contractants désignés : **AT BE CH DE ES FR GB GR IT LI NL SE**

(56) Documents cités :
**DE-A- 2 308 678**
**DE-A- 3 701 044**
**CHEMICAL ABSTRACTS, vol. 92, no. 12, mars 1980, page 335, résumé no. 99441d, Columbus, Ohio, US; & JP-A-79 110 337(NONOGAWA SHOJI Y.K.) 29-08-1979**
**Merck Index, 9th Ed., p. 9347**
**Lange's, Handbook of Chemistry, 11th Ed., Tables 5-8 + page 1642**

(73) Titulaire : **L'OREAL**
**14, Rue Royale**
**F-75008 Paris (FR)**

(72) Inventeur : **Grollier, Jean-François**
**16bis Boulevard Morland**
**F-75004 Paris (FR)**
Inventeur : **Bosq, Marie Françoise**
**81, Boulevard Maréchal Leclerc**
**F-92110 Clichy (FR)**
Inventeur : **Cotteret, Jean**
**15, Allée des Meuniers**
**F-78480 Verneuil-sur-Seine (FR)**
Inventeur : **De Labbey, Arnaud**
**9, Rue Charles Dordain**
**F-93600 Aulnay-sous-Bois (FR)**

(74) Mandataire : **Casalonga, Axel et al**
**BUREAU D.A. CASALONGA - JOSSE**
**Morassistrasse 8**
**W-8000 München 5 (DE)**

EP 0 337 853 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à un procédé de conservation du pouvoir tinctorial du 5,6-dihydroxyindole et de certains dérivés en milieu aqueux, aux compositions tinctoriales aqueuses à base de 5,6-dihydroxyindole et de ses dérivés présentant des propriétés de conservation du pouvoir tinctorial améliorées et au procédé de teinture les mettant en oeuvre.

La couleur des cheveux, de la peau et des poils en particulier d'origine humaine provient principalement des pigments mélaniques secrétés par les mélanocytes. Ces pigments, d'origine naturelle, comprennent en particulier des pigments qu'on appelle des eumélanines. Leur biosynthèse naturelle s'effectue en plusieurs étapes par polymérisation des produits d'oxydation d'un acide aminé : la tyrosine, et l'un de ces produits d'oxydation est le 5,6-dihydroxyindole qui polymérise à son tour en eumélanine.

On a déjà proposé dans le passé de repigmenter les cheveux humains avec du 5,6-dihydroxyindole en utilisant plus particulièrement des compositions aqueuses de 5,6-dihydroxyindole. Il est possible grâce à de telles compositions de teindre les cheveux, cette teinture pouvant être effectuée en plusieurs fois ou de façon progressive en repigmentant le cheveu grâce à ce colorant dans des nuances claires par une application du produit et dans une nuance de plus en plus soutenue par superposition des applications.

Ces compositions sont généralement ajustées à un pH compris entre 5 et 10 et plus particulièrement entre 7 et 9. La demanderesse a cependant constaté que la coloration des cheveux pouvait être altérée à la suite du stockage de la composition tinctoriale à base de 5,6-dihydroxyindole en milieu aqueux. On constate en effet qu'après plusieurs semaines ou mois de stockage de la composition tintoriale, les teintes obtenues sur les cheveux sont modifiées ou altérées par rapport à la teinte initiale.

La demanderesse a découvert, que de façon surprenante, ce problème de conservation du pouvoir et des caractéristiques tinctoriales du 5,6-dihydroxyindole et de ses dérivés en milieu aqueux pouvait être résolu grâce à l'utilisation, dans le milieu aquex, à titre d'agent permettant d'ajuster le pH, d'un agent à deux composants non complexants du colorant indolique.

La présente invention a donc pour object un procédé de conservation du pouvoir tinctorial du 5,6-dihydroxyindole et de ses dérivés en milieu aqueux par l'utilisation d'un agent régulateur de pH à deux composants non inhibiteurs du pouvoir tinctorial du colorant indolique.

Un autre objet de l'invention est constitué par la composition tinctoriale à base de 5,6-dihydroxyindole et/ou de ses dérivés contenant un tel agent régulateur de pH et au procédé de teinture la mettant en oeuvre.

D'autres objets de l'invention apparaîtront à la lecture de la description et des exemples qui suivent.

Le procédé de conservation du pouvoir tinctorial du 5,6-dihydroxyindole et/ou de ses dérivés répondant à la formule (I) :

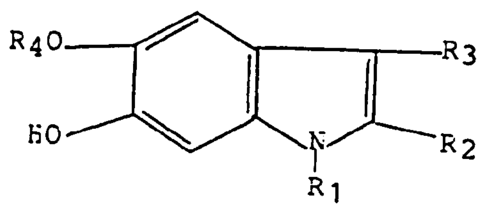

(I)

dans laquelle $R_1$, $R_2$, $R_3$, $R_4$, identiques ou différents, désignent hydrogène ou alkyle inférieur en $C_1$-$C_4$, $R_1$, $R_2$ et $R_3$ désignant hydrogène lorsque $R_4$ désigne alkyle en milieu aqueux, conforme à l'invention, est essentiellement caractérisé par le fait que l'on utilise pour ajuster le pH à une valeur $pH_C$ comprise entre 5 et 10 et de préférence entre 7 et 9, un agent régulateur de pH comportant deux composants (A) et (B), le composant (A) présentant une constante de dissociation $K_{CA}$ en solution aqueuse à 25°C, telle que $pk_{CA}$, soit

$$0 < pk_{CA} - pH_C \leqq 2{,}5$$

ou étant constitué par la trielhanolamine

le composant (B) ayant une constante de dissociation $K_{CB}$ en solution aquese à 25°C, telle que $pk_{CB}$, soit :

$$2 < pk_{CB} < pH_C$$

les composants (A) et (B) n'inhibant pas le pouvoir tinctorial du colorant indolique.

Dans la formule (I), alkyle désigne de préférence méthyle et $R_1$, $R_2$, $R_3$ ne désignent pas simultanément alkyle.

Les composés de formule (I) préférés sont choisis parmi le 5,6-dihydroxyindole, le 2-méthyl 5,6-dihydroxyindole, le 3-méthyl 5,6-dihydroxyindole, le 1-méthyl 5,6-dihydroxyindole, le 2,3-diméthyl 5,6-dihydroxyindole, le 5-méthoxy 6-hydroxyindole.

Une forme de réalisation préférée de l'invention consiste à utiliser le composant (A) dans des proportions telles que le rapport en poids du composant (A) au composant (B) soit supérieur à 1 et que le rapport molaire du composant (A) au composant (B) soit supérieur ou égal à 2 et inférieur à 15.

L'agent régulateur de pH à deux composants, utilisé conformément à l'invention, est utilisé dans des proportions comprises de préférence entre $5.10^{-3}$ et $50.10^{-3}$ moles pour 100 g de composition tinctoriale.

Les composants A et B plus particulièrement préférés dans le procédé conforme à l'invention, sont choisis parmi les couples suivants : triéthanolamine ($pk_{CA}$: 7,7)/Acide tartrique ($pk_{CB}$ : 3,2);Phosphate acide dipotassique ($pk_{CA}$ : 7,2)/Phosphate diacide de potassium ($pk_{CB}$ : 2,1).

Les compositions tinctoriales pour fibres kératiniques,,en particulier pour cheveux humains, qui constituent un autre objet de la présente invention, sont plus particulièrement caractérisées par le fait qu'elles contiennent dans un milieu aqueux approprié pour la teinture, au moins du 5,6-dihydroxyindole et/ou un de ses dérivés de formule (I) et un agent régulateur de pH permettant d'obtenir un $pH_C$ ayant une valeur comprise entre 5 et 10 et de préférence entre 7 et 9, cet agent régulateur de pH comportant deux composants (A) et (B) non complexants du colorant indolique et répondant à la définition indiquée ci-dessus.

Dans les compositions conformes à l'invention, le 5,6-dihydroxyindole et/ou ses dérivés de formule (I) est (sont) de préférence utilisé(s) dans des proportions comprises entre 0,01 et 5% en poids et de préférence entre 0,03 et 3% en poids par rapport au poids total de la composition. Les proportions particulièrement préférées sont les proportions comprises entre 0,2 et 1% en poids et plus particulièrement entre 0,3 et 0,9% en poids par rapport au poids total de la composition.

Ces compositions peuvent contenir des adjuvants appropriés pour la teinture bien connus dans les compositions tinctoriales et dans le cas où ces compositions sont destinées à être appliquées sur les cheveux, ces adjuvants sont cosmétiquement acceptables. Elles peuvent contenir plus particulièrement des solvants organiques dans les proportions de 0,5 à 50% en poids et de préférence de 2 à 20% en poids par rapport au poids total de la composition choisis plus particulièrement parmi les alcools inférieurs en $C_1$-$C_4$ tels que l'alcool éthylique, l'alcool propylique, l'alcool isopropylique, l'alcool tert.butylique, l'éthylène glycol, le propylène glycol, les éthers monométhylique, monoéthylique et monobutylique de l'éthylène glycol, l'acétate de monoéthyléther de l'éthylène glycol, les monométhyléthers du propylène glycol et du dipropylène glycol et le lactate de méthyle. Les solvants préférés sont choisis plus particulièrement parmi l'alcool éthylique et le propylène glycol.

Ces compositions peuvent être épaissies avec des agents épaississants utilisés de préférence dans des proportions en poids comprises entre 0,1 et 5% en particulier entre 0,5 et 3% par rapport au poids total de la composition. Ces épaississants sont plus particulièrement choisis parmi l'alginate de sodium, la gomme arabique, la gomme de guar, les hétérobiopolysaccharides comme la gomme de xanthane ou les scléroglucanes, les dérivés de cellulose tels que la méthylcellulose, l'hydroxyéthylcellulose, l'hydroxypropylméthylcellulose, le sel de sodium de la carboxyméthycellulose et les polymères d'acide acrylique ou encore des agents épaississants minéraux tels que la bentonite.

Ces compositions peuvent également renfermer des agents tensio-actifs anioniques, cationiques, non ioniques, amphotères ou leurs mélanges. Ces agents tensio-actifs sont de préférence utilisés dans des proportions comprises entre 0,1 et 50% en poids par rapport au poids total de la composition et avantageusement entre 1 et 20% en poids.

L'invention a également pour objet un procédé de teinture mettant en oeuvre une telle composition stabilisée dans le temps. Ce procédé consiste à appliquer la composition telle que définie ci-dessus sur des fibres kératiniques, et en particulier sur les cheveux humains, pendant un temps de pose de 1 mn à 40 mn, à faire suivre cette application par un rinçage, un shampooing éventuel, un nouveau rinçage et un séchage.

L'une des formes de réalisation préférée consiste à procéder à une teinture dite progressive consistant à appliquer plusieurs fois et de façon successive une composition tinctoriale telle que définie ci-dessus jusqu'à ce que l'on obtienne la nuance souhaitée.

Les exemples suivants sont destinés à illustrer l'invention.

## EXEMPLE 1

On procède à la coloration de cheveux naturels à 90% de blancs en appliquant sur les cheveux la composition suivante :

- 5,6-dihydroxyindole 0,5 g
- Alcool éthylique 10 g
- Gomme de Xanthane vendue sous la dénomination RHODOPOL SC par la société RHONE POULENC 2 g
- Alkyléther de glucoside de formule :

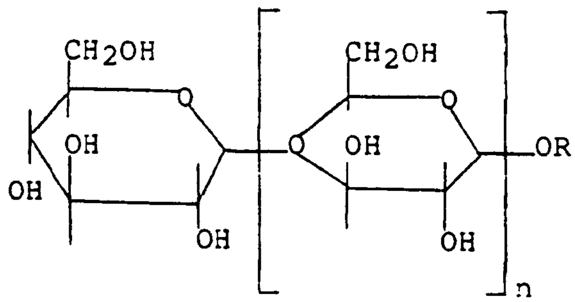

($n$ = 1 à 5, R désigne alkyle en $C_8$-$C_{10}$) vendu à 60% de matière active (MA) sous la dénomination TRITON CG 110 par la société ROHM AND HAAS 2,1 g MA
- Triéthanolamine 4 g
- Acide tartrique 0,3 g
- pH : 8,5
- Conservateur qs
- Eau qsp 100 g

On laisse poser 10 minutes. On effectue un shampooing conventionnel puis on sèche les cheveux.

Après trois applications de cette composition, les cheveux sont teints en gris moyen naturel, de la même façon qu'on teint les cheveux avec une composition identique, mais dont le pH de 8,5 a été ajusté uniquement avec la triéthanolamine, c'est-à-dire sans acide tartrique.

Après deux mois à 25°C et 45°C, la coloration obtenue à partir de la première composition suivant l'invention n'est pas altérée, tandis que celle obtenue avec la seconde composition a subi respectivement à 25 et 45°C une nette altération et très nette altération.

**EXEMPLE 2**

On procède à la coloration de cheveux naturels à 90% de blancs en appliquant sur les cheveux la composition suivante :

| | | |
|---|---|---|
| - 5,6-dihydroxyindole | | 0,5 g |
| - Alcool éthylique | | 10 g |
| - Gomme de Xanthane vendue sous la dénomination RHODOPOL SC par la société RHONE POULENC | | 2 g |
| - Alkyléther de glucoside de formule : | | |

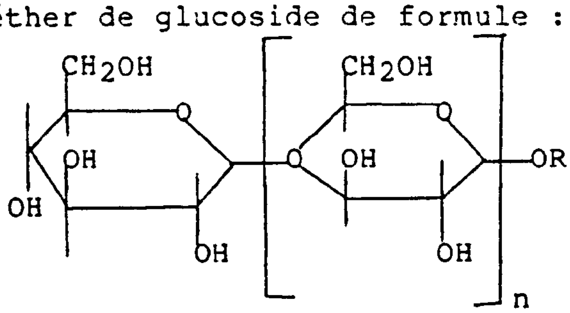

| | | |
|---|---|---|
| (n = 1 à 5, R désigne alkyle en $C_8$-$C_{10}$) vendu à 60% de matière active (MA) sous la dénomination TRITON CG 110 par la société ROHM AND HAAS | | 2,1 g MA |
| - Phosphate acide dipotassique | | 0,87 g |
| - Phosphate diacide de potassium | | 0,30 g |
| - pH : 7,1 | | |
| - Conservateur | qs | |
| - Eau | qsp | 100 g |

On laisser poser 10 minutes. On effectue un shampooing puis on sèche les cheveux.

Après trois applications de cette composition, les cheveux sont teints en gris beige clair.

## EXEMPLE 3

On procède à la coloration de cheveux naturels à 90% de blancs en appliquant sur les cheveux la composition suivante :

| | | | |
|---|---|---|---|
| - 5,6-dihydroxyindole | | 0,8 | g |
| - Alcool éthylique | | 10 | g |
| - Gomme de Xanthane vendue sous la dénomination RHODOPOL SC par la société RHONE POULENC | | 2 | g |
| - Alkyléther de glucoside de formule : 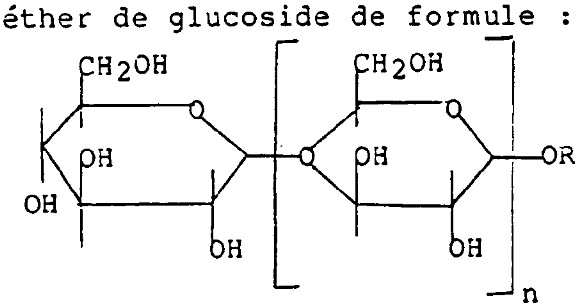 (n = 1 à 5, R désigne alkyle en $C_8$-$C_{10}$) vendu à 60% de matière active (MA) sous la dénomination TRITON CG 110 par la société ROHM AND HAAS | | 2,1 | g MA |
| - Triéthanolamine | | 4 | g |
| - Acide tartrique | | 0,3 | g |
| - pH : 8,5 | | | |
| - Conservateur | qs | | |
| - Eau | qsp | 100 | g |

On laisse poser 10 minutes. On effectue un shampooing puis on sèche les cheveux.

Après trois applications de cette composition, les cheveux sont teints en gris moyen.

## EXEMPLE 4

On procède à la coloration de cheveux naturels à 90% de blancs en appliquant sur les cheveux la composition suivante :

| | | |
|---|---|---|
| - 3-méthyl 5,6-dihydroxyindole | | 0,2 g |
| - Alcool éthylique | | 10 g |
| - Gomme de Xanthane vendue sous la dénomination RHODOPOL SC par la société RHONE POULENC | | 2 g |
| - Alkyléther de glucoside de formule : | | |

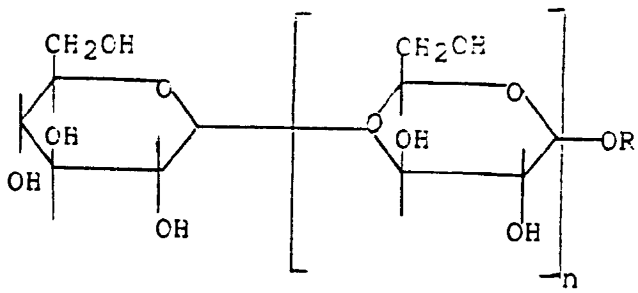

| | | |
|---|---|---|
| (n = 1 à 5, R désigne alkyle en $C_8$-$C_{10}$) vendu à 60% de matière active (MA) sous la dénomination TRITON CG 110 par la société ROHM AND HAAS | | 2,1 g MA |
| - Triéthanolamine | | 4 g |
| - Acide tartrique | | 0,3 g |
| - pH : 8,5 | | |
| - Conservateur | qs | |
| - Eau | qsp | 100 g |

On laisse poser 10 minutes. On effectue un shampooing conventionnel puis on sèche les cheveux. Après trois applications, les cheveux sont teints en cendré bleu.

## EXEMPLE 5

On procède à la coloration de cheveux naturels à 90% de blancs en appliquant sur les cheveux la composition-suivante :

| | | |
|---|---|---|
| - 3-méthyl 5,6-dihydroxyindole | | 0,5 g |
| - Alcool éthylique | | 10 g |
| - Gomme de Xanthane vendue sous la dénomination RHODOPOL SC par la société RHONE POULENC | | 2 g |
| - Alkyléther de glucoside de formule : | | |

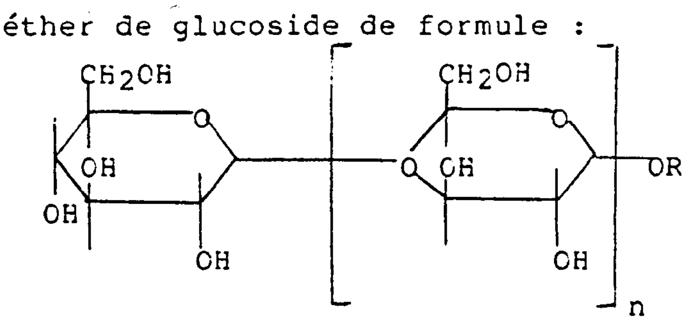

| | | |
|---|---|---|
| (n : 1 à 5, R désigne alkyle en $C_8$-$C_{10}$) vendu à 60% de matière active (MA) sous la dénomination TRITON CG 110 par la société ROHM AND HAAS | | 2,1 g MA |
| - Triéthanolamine | | 4 g |
| - Acide tartrique | | 0,3 g |
| - pH : 8,5 | | |
| - Conservateur | qs | |
| - Eau | qsp | 100 g |

On laisse poser 10 minutes. On effectue un shampooing conventionnel puis on sèche les cheveux.

Après une application, les cheveux sont teints en une nuance bleu cendré qui se renforce après plusieurs applications.

**EXEMPLE 6**

On procède à la coloration de cheveux naturels à 90% de blancs en appliquant sur les cheveux la composition suivante :

- 2-méthyl 5,6-dihydroxyindole 0,5 g
- Alcool éthylique 10 g
- Gomme de Xanthane vendue sous la dénomination RHODOPOL SC par la société RHONE POULENC 2 g
- Alkyléther de glucoside de formule :

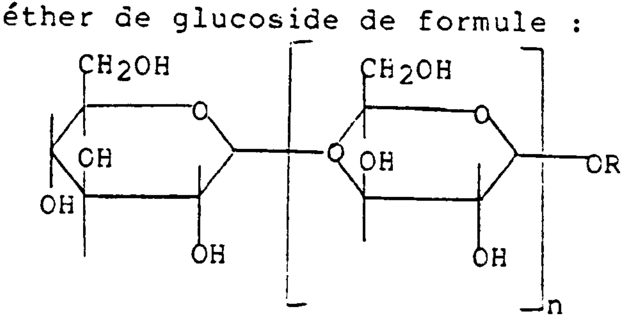

(n = 1 à 5, R désigne alkyle en $C_8$-$C_{10}$) vendu à 60% de matière active (MA) sous la dénomination TRITON CG 110 par la société ROHM AND HAAS 2,1 g MA
- Triéthanolamine 4 g
- Acide tartrique 0,3 g
- pH : 8,5
- Conservateur qs
- Eau qsp 100 g

On laisse poser 10 minutes. On effectue un shampooing conventionnel puis on sèche les cheveux. Après une application de cette composition, les cheveux sont teints en blond clair beige.

**EXEMPLE 7**

On procède à la coloration de cheveux naturels à 90% de blancs en appliquant sur les cheveux la composition suivante :

| | | |
|---|---|---|
| - 2,3-diméthyl 5,6-dihydroxyindole | | 0,5 g |
| - Alcool éthylique | | 10 g |
| - Gomme de Xanthane vendue sous la dénomination RHODOPOL SC par la société RHONE POULENC | | 2 g |
| - Alkyléther de glucoside de formule : | | |

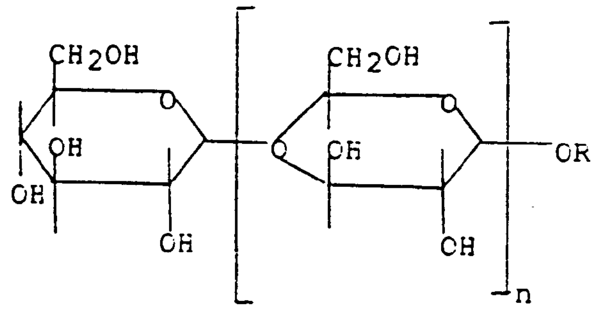

| | | |
|---|---|---|
| ($n$ = 1 à 5, R désigne alkyle en $C_8$-$C_{10}$) vendu à 60% de matière active (MA) sous la dénomination TRITON CG 110 par la société ROHM AND HAAS | | 2,1 g MA |
| - Triéthanolamine | | 4 g |
| - Acide tartrique | | 0,3 g |
| - pH : 8,5 | | |
| - Conservateur | qs | |
| - Eau | qsp | 100 g |

On laisse poser 10 minutes. On effectue un shampooing conventionnel puis on sèche les cheveux. Après une application de cette composition, les cheveux sont teints en blond clair cendré.

## Revendications

**Revendications pour les Etats contractants suivants : DE, GB, FR, IT, NL, SE, CH, BE, AT**

1. Procédé de conservation du pouvoir tinctorial du 5,6-dihydroxy indole et/ou à la formule (I) :

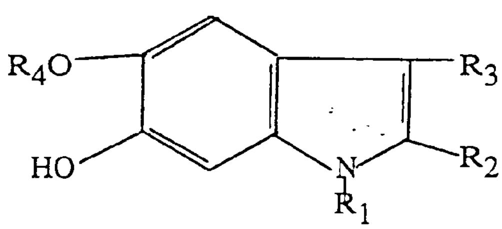

(I)

dans laquelle $R_1$, $R_2$, $R_3$, $R_4$, identiques ou différents, désignent hydrogène ou alkyle inférieur en $C_1$-$C_4$, $R_1$, $R_2$ et $R_3$ désignant hydrogène lorsque $R_4$ désigne alkyle, en milieu aqueux, caractérisé par le fait que l'on ajuste le pH du milieu aqueux à une valeur $pH_C$ comprise entre 5 et 10 en utilisant un agent régulateur de pH à deux composants (A) et (B), le composant (A) étant choisi parmi les agents régulateurs de pH ayant une constante de dissociation $K_{CA}$, en solution aqueuse à 25°C, telle que le $pk_{CA}$, soit :

$$O < pk_{CA} - pH_C \leqq 2,5$$

et le composant (B) étant choisi parmi les agents régulateurs de pH ayant une constante de dissociation $K_{CB}$, en solution aqueuse à 25°C, telle que le $pk_{CB}$, soit :

$$2 < pk_{CB} < pH_C.$$

2. Procédé selon la revendication 1, caractérisé par le fait que le composé de formule (I) est le 5,6-dihydroxyindole.

3. Procédé selon la revendication 1, caractérisé par le fait que le composé de formule (I) est choisi parmi le 2-méthyl 5,6-dihydroxy indole, le 3-méthyl 5,6-dihydroxyindole, le 1-méthyl 5,6-dihydroxy idole, le 2,3-diméthyl 5,6-dihydroxyindole, le 5-méthoxy 6-hydroxy indole.

4. Procédé de conservation du pouvoir tinctorial du 5,6-dihydroxy indole et/ou de ses dérivés répondant à la formule (I) :

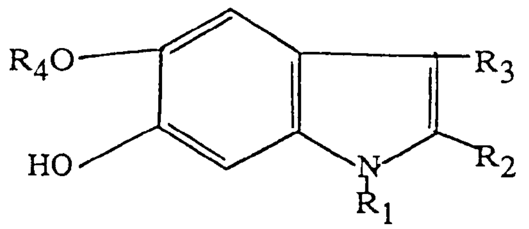

(I)

dans laquelle $R_1$, $R_2$, $R_3$, $R_4$, identiques ou différents, désignent hydrogène ou alkyle inférieur en $C_1$-$C_4$, $R_1$, $R_2$ et $R_3$ désignant hydrogène lorsque $R_4$ désigne alkyle,en milieu aqueux, caractérisé par le fait que l'on ajuste le pH du milieu aqueux à une valeur $pH_C$ comprise entre 5 et 10 en utilisant un agent régulateur de pH à deux composants (A) et (B), le composant (A) étant la triéthanolamine et le composant (B) étant choisi parmi les agents régulateurs de pH ayant une constante de dissociation $K_{CB}$, en solution aqueuse à 25°C, telle que le $pk_{CB}$, soit :

$$2 < pk_{CB} < pH_C.$$

5. Procédé selon la revendication 4, caractérisé par le fait que le composant (B) est constitué par l'acide tartrique ayant une valeur $pk_{CB}$ de 3,2.

6. Procédé selon la revendication 4 ou 5, caractérisé par le fait que le composé de formule (I) est le 5,6-dihydroxyindole.

7. Procédé selon la revendication 4 ou 5, caractérisé par le fait que le composé de formule (I) est choisi parmi le 2-méthyl 5,6-dihydroxyindole, le 3-méthyl 5,6-dihydroxyindole, le 1-méthyl 5,6-dihydroxyindole, le 2,3-diméthyl 5,6-dihydroxyindole, le 5-méthoxy 6- hydroxyindole.

8. Procédé selon l'une quelconque des revendications 1 à 3, caractérisé par le fait que le composant (A) est constitué par le phosphate acide dipotassique ayant une valeur de $pk_{CA}$ de 7,2 et que le composant (B) est constitué par le phosphate diacide de potassium ayant une valeur $pk_{CB}$ de 2,1.

9. Procédé selon l'une quelconque des revendications 1 à 8, caractérisé par le fait que le rapport en poids du composant (A) au composant (B) est supérieur à 1 et que le rapport molaire du composait (A) au composant (B) est supérieur ou égal à 2 et inférieur à 15.

10. Procédé selon l'une quelconque des revendications 1 à 9, caractérisé par le fait que l'agent régulateur de pH à deux composants (A) et (B) est présent dans des proportions comprises entre $5.10^{-3}$ et $50.10^{-3}$ moles pour 100 g de composition tinctoriale.

11. Procédé selon l'une quelconque des revendications 1 à 10, caractérisé par le fait que le pH du milieu a une valeur $pH_C$ comprise entre 7 et 9.

12. Composition tinctoriale pour fibres kératiniques, en particulier pour cheveux humains, caractérisée par le fait qu'elle contient dans un milieu aqueux au moins du 5,6-dihydroxyindole et/ou ses dérivés de formule (I) :

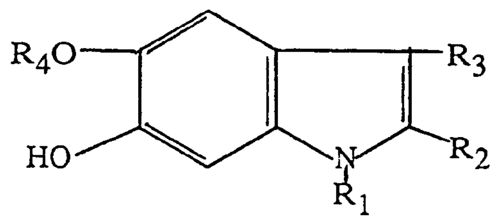

(I)

dans laquelle $R_1$, $R_2$, $R_3$, $R_4$, identiques ou différents, désignent hydrogène ou alkyle inférieur en $C_1$-$C_4$, $R_1$, $R_2$ et $R_3$ désignant hydrogène lorsque $R_4$ désigne alkyle, et un agent régulateur de pH permettant d'ajuster le pH à une valeur $pH_C$ comprise entre 5 et 10, constitué par un agent régulateur de pH à deux composants (A) et (B), le composant (A) ayant une constante de dissociation $K_{CA}$, en solution aqueuse à 25°C, telle que $pk_{CA}$, soit :

$$O < pk_{CA} - pH_C \leqq 2,5$$

et le composant (B) ayant une constante de dissociation $K_{CB}$, en solution aqueuse à 25°C, telle que $pk_{CB}$, soit :

$$2 < pk_{CB} < pH_C$$

les composants (A) et (B) n'inhibant pas le pouvoir tinctorial du colorant.

13. Composition selon la revendication 12, caractérisée par le fait que le composé de formule (I) est le 5,6-dihydroxyindole.

14. Composition selon la revendication 12, caractérisée par le fait que le composé de formule (I) est choisi parmi le 2-méthyl 5,6-dihydroxyindole, le 3-méthyl 5,6-dihydroxyindole, le 1-méthyl 5,6-dihydroxyindole, le 2,3-diméthyl 5,6-dihydroxyindole, le 5-méthoxy 6-hydroxyindole.

15. Composition tinctoriale pour fibres kératiniques, en particulier pour cheveux humains, caractérisée par le fait qu'elle contient dans un milieu aqueux au moins du 5,6-dihydroxyindole et/ou ses dérivés de formule (I) :

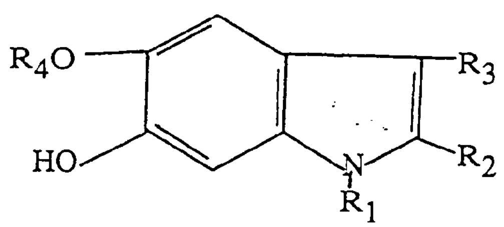

(I)

dans laquelle $R_1$, $R_2$, $R_3$, $R_4$, identiques ou différents, désignent hydrogène ou alkyle inférieur en $C_1$-$C_4$, $R_1$, $R_2$ et $R_3$ désignant hydrogène lorsque $R_4$ désigne alkyle, et un agent régulateur de pH permettant d'ajuster le pH à une valeur $pH_C$ comprise entre 5 et 10,constitué par un agent régulateur de pH à deux composants (A) et (B), le composant (A) étant constitué par la triéthanolamie et le composant (B) ayant une constante de dissociation $K_{CB}$, en solution aqueuse à 25°C, telle que $pk_{CB}$, soit :

$$2 < pk_{CB} < pH_C,$$

les composants (A) et (B) n'inhibant pas le pouvoir tinctorial du colorant

16. Composition selon la revendication 15, caractérisée par le fait que le composé de formule (I) est le 5,6-dihydroxyindole.

17. Composition selon la revendication 15, caractérisée par le fait que le composé de formule (I) est choisi parmi le 2-méthyl 5,6-dihydroxyindole, le 3-méthyl 5,6-dihydroxyindole, le 1-méthyl 5,6-dihydroxyindole, le 2,3-diméthyl 5,6-dihydroxyindole, le 5-méthoxy 6-hydroxyindole.

18. Composition selon l'une quelconque des revendications 15 à 17, caractérisée par le fait que le composant (B) est l'acide tartrique ayant une valeur $pk_{CB}$ de 3,2.

19. Composition selon l'une quelconque des revendications 12 à 18, caractérisée par le fait que les composants (A) et (B) sont présents dans des proportions en poids tels que le rapport du composant (A) au composant (B) soit supérieur à 1 et dans des proportions molaires telles que le rapport du composant (A) au composant (B) est supérieur ou égal à 2 et inférieur à 15.

20. Composition selon l'une quelconque des revendications 12 à 19, caractérisée par le fait que le 5,6-dihydroxyindole et/ou ses dérivés de formule (I) est (sont) présent(s) dans des proportions comprises entre 0,01 et 5% en poids et de préférence entre 0,2 et 1% en poids par rapport au poids total de la composition.

21. Composition selon l'une quelconque des revendications 12 à 20, caractérisée par le fait que la composition contient un solvant dans des proportions comprises entre 0,5 et 50% en poids, de préférence de 2 à 20% en poids, choisi parmi les alcools inférieurs en $C_1$-$C_4$, l'éthylèneglycol ou le propylèneglycol, les éthers monométhylique, monoéthylique et monobutylique de l'éthylèneglycol, l'acétate du monoéthyléther de l'éthylèneglycol, les monométhyléthers du propylèneglycol et du dipropylèneglycol et le lactate de méthyle.

22. Composition selon l'une quelconque des revendications 12 à 21, caractérisée par le fait que la composition est épaissie à l'aide d'agents épaississants utilisés dans des proportions en poids comprises entre 0,1 et 5% et de préférence entre 0,5 et 3% par rapport au poids total de la composition.

23. Composition selon l'une quelconque des revendications 12 à 22, caractérisée par le fait qu'elle contient également des agents tensio-actifs cationiques, non-ioniques, anioniques, amphotères ou leurs mélanges, dans des proportions comprises entre 0,1 et 50% en poids par rapport au poids total de la composition.

24. Procédé de teinture des fibres kératiniques, en particulier des cheveux hunmains, caractérisé par le fait que l'on applique la composition telle que définie dans l'une quelconque des revendications 12 à 23 sur lesdites fibres et qu'après un temps de pose compris entre 1 minute et 40 minutes, on rince lesdites fibres.

25. Procédé de teinture des fibres kératiniques, en particulier des cheveux, caractérisé par le fait que l'on procède à une teinture progressive des cheveux en appliquant plusieurs fois et de façon successive, la composition telle que définie dans l'une quelconque des revendications 12 à 23.

**Revendications pour les Etats contractants suivants : ES, GR**

1. Procédé de conservation du pouvoir tinctorial du 5,6-dihydroxy indole et/ou de ses dérivés répondant à la formule (I) :

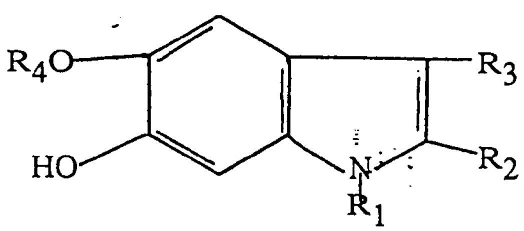

(I)

dans laquelle $R_1$, $R_2$, $R_3$, $R_4$, identiques ou différents, désignent hydrogène ou alkyle inférieur en $C_1$ -$C_4$, $R_1$, $R_2$ et $R_3$ désignant hydrogène lorsque $R_4$ désigne alkyle en milieu aqueux, caractérisé par le fait que l'on ajuste le pH du milieu aqueux à une valeur $pH_C$ comprise entre 5 et 10 en utilisant un agent régulateur de pH à deux composants (A) et (B), le composant (A) étant choisi parmi les agents régulateurs de pH ayant une constante de dissociation $K_{CA}$, en solution aqueuse à 25°C, telle que le $pk_{CA}$, soit :

$$O < pk_{CA} - pH_C \leqq 2{,}5$$

et le composant (B) étant choisi parmi les agents régulateurs de pH ayant une constante de dissociation $K_{CB}$, en solution aqueuse à 25°C, telle que le $pk_{CB}$, soit :

$$2 < pk_{CB} < pH_C.$$

2. Procédé selon la revendication 1, caractérisé par le fait que le composé de formule (I) est le 5,6-dihydroxyindole.

3. Procédé selon la revendication 1, caractérisé par le fait que le composé de formule (I) est choisi parmi le 2-méthyl 5,6-dihydroxy indole, le 3-méthyl 5,6-dihydroxyindole, le 1-méthyl 5,6-dihydroxy indole, le 2,3-diméthyl 5,6-dihydroxyindole, le 5-méthoxy 6- hydroxy indole.

4. Procédé de conservation du pouvoir tinctorial du 5,6-dihydroxy indole et/ou de ses dérivés répondant à la formule (I) :

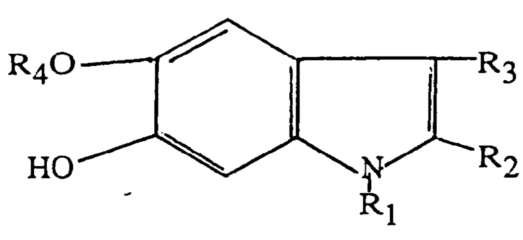

(I)

dans laquelle $R_1$, $R_2$, $R_3$, $R_4$, identiques ou différents, désignent hydrogène ou alkyle inférieur en $C_1$-$C_4$, $R_1$, $R_2$ et $R_3$ désignant hydrogène lorsque $R_4$ désigne alkyle en milieu aqueux, caractérisé par le fait que l'on ajuste

le pH du milieu aqueux à une valeur $pH_C$ comprise entre 5 et 10 en utilisant un agent régulateur de pH à deux composants (A) et (B), le composant (A) étant la triéthanolamine et le composant (B) étant choisi parmi les agents régulateurs de pH ayant une constante de dissociation $K_{CB}$, en solution aqueuse à 25°C, telle que le $pk_{CB}$, soit :

$$2 < pk_{CB} < pH_C.$$

5. Procédé selon la revendication 4, caractérisé par le fait que le composant (B) est constitué par l'acide tartrique ayant une valeur $pk_{CB}$ de 3,2.

6. Procédé selon la revendication 4 ou 5, caractérisé par le fait que le composé de formule (I) est le 5,6-dihydroxyindole.

7. Procédé selon la revendication 4 ou 5, caractérisé par le fait que le composé de formule (I) est choisi parmi le 2-méthyl 5,6-dihydroxyindole, le 3-méthyl 5,6-dihydroxyindole, le 1-méthyl 5,6-dihydroxyindole, le 2,3-diméthyl 5,6-dihydroxyidole, le 5-méthoxy 6-hydroxyindole.

8. Procédé selon l'une quelconque des revendications 1 à 3, caractérisé par le fait que le composant (A) est constitué par le phosphate acide dipotassique ayant une valeur de $pk_{CA}$ de 7,2 et que le composant (B) est constitué par le phosphate diacide de potassium ayant une valeur $pk_{CB}$ de 2,1.

9. Procédé selon l'une quelconque des revendications 1 à 8, caractérisé par le fait que le rapport en poids du composant (A) au composant (B) est supérieur à 1 et que le rapport molaire du composant (A) au composant (B) est supérieur ou égal à 2 et inférieur à 15.

10. Procédé selon l'une quelconque des revendications 1 à 9, caractérisé par le fait que l'agent régulateur de pH à deux composants (A) et (B) est présent dans des proportions comprises entre $5.10^{-3}$ et $50.10^{-3}$ moles pour 100 g de composition tinctoriale.

11. Procédé selon l'une quelconque des revendications 1 à 10, caractérisé par le fait que le pH du milieu a une valeur $pH_C$ comprise entre 7 et 9.

12. Composition tinctoriale pour fibres kératiniques, en particulier pour cheveux humains, caractérisée par le fait qu'elle contient dans un milieu aqueux au moins du 5,6-dihydroxyindole et/ou ses dérivés de formule (I) :

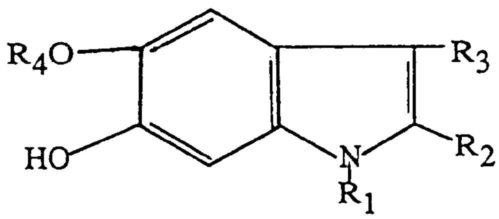

(I)

dans laquelle $R_1$, $R_2$, $R_3$, $R_4$, identiques ou différents, désignent hydrogène ou alkyle inférieur en $C_1$-$C_4$, $R_1$, $R_2$ et $R_3$ désignant hydrogène lorsque $R_4$ désigne alkyle, et un agent régulateur de pH permettant d'ajuster le pH à une valeur $pH_C$ comprise entre 5 et 10, constitué par un agent régulateur de pH à deux composants (A) et (B), le composant (A) ayant une constante de dissociation $K_{CA}$, en solution aqueuse à 25°C, telle que $pk_{CA}$, soit :

$$O < pk_{CA} - pH_C \leqq 2,5$$

et le composant (B) ayant une constante de dissociation $K_{CB}$, en solution aqueuse à 25°C, telle que $pk_{CB}$, soit :

$$2 < pk_{CB} < pH_C,$$

les composants (A) et (B) n'inhibant pas le pouvoir tinctorial du colorant.

13. Composition selon la revendication 12, caractérisée par le fait que le composé de formule (I) est le 5,6-dihydroxyindole.

14. Composition selon la revendication 12, caractérisée par le fait que le composé de formule (I) est choisi parmi le 2-méthyl 5,6-dihydroxyindole, le 3-méthyl 5,6-dihydroxyindole, le 1-méthyl 5,6-dihydroxyindole, le 2,3-diméthyl 5,6-dihydroxyindole, le 5-méthoxy 6-hydroxyindole.

15. Composition tinctoriale pour fibres kératiniques, en particulier pour cheveux humains, caractérisée par le fait qu'elle contient dans un milieu aqueux au moins du 5,6-dihydroxyindole et/ou ses dérivés de formule (I) :

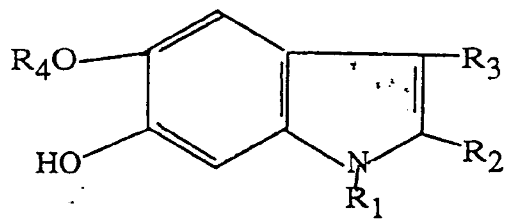

(I)

dans laquelle $R_1$, $R_2$, $R_3$, $R_4$, identiques ou différents, désignent hydrogène ou alkyle inférieur en $C_1$-$C_4$, $R_1$, $R_2$ et $R_3$ désignant hydrogène lorsque $R_4$ désigne alkyle, et un agent régulateur de pH permettant d'ajuster le pH à une valeur $pH_C$ comprise entre 5 et 10, constitué par un agent régulateur de pH à deux composants (A) et (B), le composant (A) étant constitué par la triéthanolamine et le composant (B) ayant une constante de dissociation $K_{CB}$, en solution aqueuse à 25°C, telle que $pk_{CB}$, soit :

$$2 < pk_{CB} < pH_C,$$

les composants (A) et (B) n'inhibant pas le pouvoir tinctorial du colorant.

16. Composition selon la revendication 15, caractérisée par le fait que le composé de formule (I) est le 5,6-dihydroxyindole.

17. Composition selon la revendication 15, caractérisée par le fait que le composé de formule (I) est choisi parmi le 2-méthyl 5,6-dihydroxyindole, le 3-méthyl 5,6-dihydroxyindole, le 1-méthyl 5,6-dihydroxyindole, le 2,3-diméthyl 5,6-dihydroxyindole, le 5-méthoxy 6-hydroxyindole.

18. Composition selon l'une quelconque des revendications 15 à 17, caractérisée par le fait que le composant (B) est l'acide tartrique ayant une valeur $pk_{CB}$ de 3,2.

19. Composition selon l'une quelconque des revendications 12 à 18, caractérisée par le fait que les composants (A) et (B) sont présents dans des proportions en poids tels que le rapport du composant (A) au composant (B) soit supérieur à 1 et dans des proportions molaires telles que le rapport du composant (A) au composant (B) est supérieur ou égal à 2 et inférieur à 15.

20. Composition selon l'une quelconque des revendications 12 à 19, caractérisée par le fait que le 5,6-dihydroxyindole et/ou ses dérivés de formule (I) est (sont) présent(s) dans des proportions comprises entre 0,01 et 5% en poids et de préférence entre 0,2 et 1% en poids par rapport au poids total de la composition.

21. Composition selon l'une quelconque des revendications 12 à 20, caractérisée par le fait que la composition contient un solvant dans des proportions comprises entre 0,5 et 50% en poids, de préférence de 2 à 20% en poids, choisi parmi les alcools inférieurs en $C_1$-$C_4$, l'éthylèneglycol ou le propylèneglycol, les éthers monométhylique, monoéthylique et monobutylique de l'éthylèneglycol, l'acétate du monoéthyléther de l'éthylèneglycol, les monométhyléthers du propylèneglycol et du dipropylèneglycol et le lactate de méthyle.

22. Composition selon l'une quelconque des revendications 12 à 21, caractérisée par le fait que la composition est épaissie à l'aide d'agents épaississants utilisés dans des proportions en poids comprises entre 0,1 et 5% et de préférence entre 0,5 et 3% par rapport au poids total de la composition.

23. Composition selon l'une quelconque des revendications 12 à 22, caractérisée par le fait qu'elle contient également des agents tensio-actifs cationiques, non-ioniques, anioniques, amphotères ou leurs mélanges, dans des proportions comprises entre 0,1 et 50% en poids par rapport au poids total de la composition.

24. Procédé de teinture des fibres kératiniques, en particulier des cheveux humains, caractérisé par le fait que l'on applique la composition telle que définie dans l'une quelconque des revendications 12 à 23 sur lesdites fibres et qu'après un temps de pose compris entre 1 minute et 40 minutes, on rince lesdites fibres.

25. Procédé de teinture des fibres kératiniques, en particulier des cheveux, caractérisé par le fait que l'on procède à une teinture progressive des cheveux en appliquant plusieurs fois et de façon successive, la composition telle que définie dans l'une quelconque des revendications 12 à 23.

26. Procédé de préparation d'une composition tinctoriale pour fibres kératiniques, en particulier pour cheveux humains, caractérisé par le fait qu'on introduit dans un milieu aqueux au moins du 5,6-dihydroxyindole et/ou ses dérivés de formule (I) :

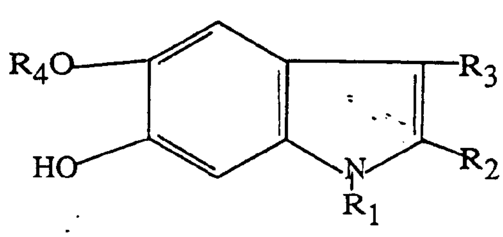

(I)

dans laquelle $R_1$, $R_2$, $R_3$, $R_4$, identiques ou différents, désignent hydrogène ou alkyle inférieur en $C_1$-$C_4$, $R_1$, $R_2$ et $R_3$ désignant hydrogène lorsque $R_4$ désigne alkyle, dans des proportions comprises entre 0,01 et 5% en poids par rapport au poids total de la composition, et un agent régulateur de pH permettant d'ajuster le pH à une valeur $pH_C$ comprise entre 5 et 10, constitué par un agent régulateur de pH à deux composants (A) et (B), le composant (A) ayant une constante de dissociation $K_{CA}$, en solution aqueuse à 25°C, telle que $pk_{CA}$, soit :

$$0 < pk_{CA} - pH_C \leqq 2,5$$

et le composant (B) ayant une constante de dissociation $K_{CB}$, en solution aqueuse à 25°C, telle que $pk_{CB}$, soit :

$$2 < pk_{CB} < pH_C$$

les composants (A) et (B) n'inhibant pas le pouvoir tinctorial du colorant.

27. Procédé de préparation d'une composition tinctoriale pour fibres kératiniques, en particulier pour cheveux humains, caractérisé par le fait qu'on introduit dans un milieu aqueux au moins du 5,6-dihydroxyindole et/ou ses dérivés de formule (I) :

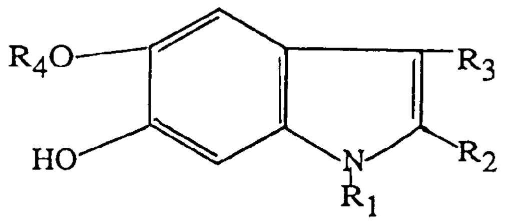

(I)

dans laquelle $R_1$, $R_2$, $R_3$, $R_4$, identiques ou différents, désignent hydrogène ou alkyle inférieur en $C_1$-$C_4$, $R_1$, $R_2$ et $R_3$ désignant hydrogène lorsque $R_4$ désigne alkyle, dans des proportions comprises entre 0,01 et 5% en poids par rapport au poids total de la composition, et un agent régulateur de pH permettant d'ajuster le pH à une valeur $pH_C$ comprise entre 5 et 10, constitué par un agent régulateur de pH à deux composants (A) et (B), le composant (A) étant la triéthanolamie et le composant (B) ayant une constante de dissociation $K_{CB}$, en solution aqueuse à 25°C, telle que $pk_{CB}$, soit :

$$2 < pk_{CB} < pH_C$$

les composants (A) et (B) n'inhibant pas le pouvoir tinctorial du colorant.

## Patentansprüche

### Patentansprüche für folgende Vertragsstaaten : DE, GB, FR, IT, NL, SE, CH, BE, AT

1. Verfahren zur Bewahrung des Färbevermögens in wässrigem Milieu von 5,6-Dihydroxyindol und/oder seiner Derivate der Formel (I):

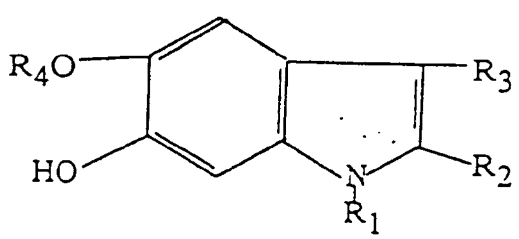

(I)

worin $R_1$, $R_2$, $R_3$, $R_4$, gleich oder verschieden, Wasserstoff oder eine $C_1$-$C_4$-Niedrigalkylgruppe bezeichnen, wobei $R_1$, $R_2$ und $R_3$ Wasserstoff bedeuten, wenn $R_4$ Alkyl bedeutet, dadurch **gekennzeichnet**, daß man den pH des wässrigen Milieus auf einen $pH_C$-Wert von 5 bis 10 einstellt, indem man ein pH-Regulierungsmittel aus 2 Bestandteilen (A) und (B) verwendet, wobei der Bestandteil (A) unter pH-Regulierungsmitteln mit einer Dissoziationskonstante $K_{CA}$, deren $pk_{CA}$-Wert in wässriger Lösung bei 25°C beträgt:

$$0 < pk_{CA} - pH_C \leqq 2,5$$

und der Bestandteil (B) unter pH-Regulierungsmitteln mit einer Dissoziationskonstante $K_{CB}$, deren $pk_{CB}$-Wert in wässriger Lösung bei 25°C beträgt:

$$2 < pk_{CB} < pH_C$$

ausgewählt sind.

2. Verfahren gemäß Anspruch 1, dadurch **gekennzeichnet**, daß die Verbindung der Formel (I) 5,6-Dihydroxyindol ist.

3. Verfahren gemäß Anspruch 1, dadurch **gekennzeichnet** , daß die Verbindung der Formel (I) unter 2-Methyl-5,6-dihydroxyindol, 3-Methyl-5,6-dihydroxyindol, 1-Methyl-5,6-dihydroxyindol, 2,3-Dimethyl-5,6-dihydroxyindol, 5-Methoxy-6-hydroxyindol ausgewählt ist.

4. Verfahren zur Bewahrung des Färbevermögens in wässrigem Milieu von 5,6-Dihydroxyindol und/oder seiner Derivate der Formel (I):

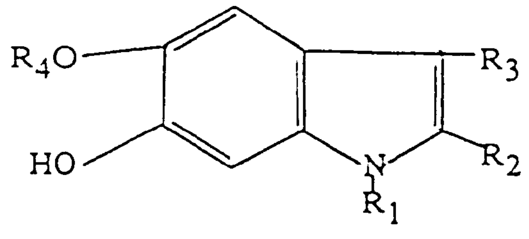

(I)

worin $R_1$, $R_2$, $R_3$, $R_4$, gleich oder verschieden, Wasserstoff oder eine $C_1$-$C_4$-Niedrigalkylgruppe bezeichnen, wobei $R_1$, $R_2$ und $R_3$ Wasserstoff bedeuten, wenn $R_4$ Alkyl bedeutet, dadurch **gekennzeichnet** , daß man den pH des wässrigen Milieus auf einen $pH_C$-Wert von 5 bis 10 einstellt, indem man ein pH-Regulierungsmittel aus 2 Bestandteilen (A) und (B) verwendet, wobei der Bestandteil (A) Triethanolamin ist und der Bestandteil (B) unter pH-Regulierungsmitteln mit einer Dissoziationskonstante $K_{CB}$ ausgewählt ist, deren $pk_{CB}$-Wert in wässriger Lösung bei 25°C:

$$2 < pk_{CB} < pH_C$$

beträgt.

5. Verfahren gemäß Anspruch 4, dadurch **gekennzeichnet** , daß der Bestandteil (B) aus Weinsäure mit einem $pk_{CB}$-Wert von 3,2 zusammengesetzt ist.

6. Verfahren gemäß Anspruch 4 oder 5, dadurch **gekennzeichnet** , daß die Verbindung der Formel (I) 5,6-Dihydroxyindol ist.

7. Verfahren gemäß Anspruch 4 oder 5, dadurch **gekennzeichnet** , daß die Verbindung der Formel (I) unter 2-Methyl-5,6-dihydroxyindol, 3-Methyl-5,6-dihydroxyindol, 1-Methyl-5,6-dihydroxyindol, 2,3-Dimethyl-5,6-dihydroxyindol, 5-Methoxy-6-hydroxyindol ausgewählt ist.

8. Verfahren gemäß jedem der Ansprüche 1 bis 3, dadurch **gekennzeichnet** , daß der Bestandteil (A) aus saurem Dikaliumphosphat mit einem $pk_{CA}$-Wert von 7,2 und der Bestandteil (B) aus Kaliumdihydrogenphosphat mit einem $pk_{CB}$-Wert von 2,1 zusammengesetzt sind.

9. Verfahren gemäß jedem der Ansprüche 1 bis 8, dadurch **gekennzeichnet** , daß das Gewichtsverhältnis des Bestandteils (A) zum Bestandteil (B) größer als 1 und das molare Verhältnis des Bestandteils (A) zum Bestandteil (B) größer oder gleich 2 und kleiner als 15 sind.

10. Verfahren gemäß jedem der Ansprüche 1 bis 9, dadurch **gekennzeichnet** , daß das pH-Regulierungsmittel aus den zwei Bestandteilen (A) und (B) in Mengenverhältnissen von $5 \times 10^{-3}$ bis $50 \times 10^{-3}$ Mol pro 100 g Färbezusammensetzung vorliegt.

11. Verfahren gemäß jedem der Ansprüche 1 bis 10, dadurch **gekennzeichnet** , daß der pH des Milieus einen $pH_C$-Wert von 7 bis 9 aufweist.

12. Färbezusammensetzung für keratinische Fasern, insbesondere menschliches Haar, dadurch **gekennzeichnet** , daß sie in einem wässrigen Milieu mindestens 5,6-Dihydroxyindol und/oder seine Derivate der Formel (I):

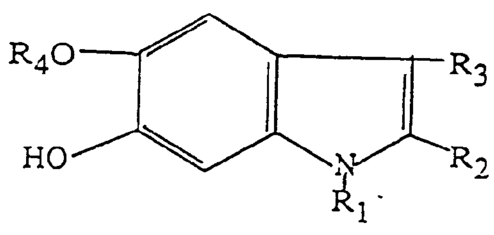

(I)

worin $R_1$, $R_2$, $R_3$, $R_4$ , leich oder verschieden, Wasserstoff oder eine $C_1$-$C_4$-Niedrigalkylgruppe bezeichnen, wobei $R_1$, $R_2$ und $R_3$ Wasserstoff bedeuten, wenn $R_4$ Alkyl bedeutet, sowie ein pH-Regulierungsmittel enthält, welches es zuläßt, den pH auf einen $pH_C$-Wert von 5 bis 10 einzustellen, und das aus einem pH-Regulierungsmittel aus 2 Bestandteilen (A) und (B) zusammengesetzt ist, wobei der Bestandteil (A) eine Dissoziationskonstante $K_{CA}$, deren $pk_{CA}$-Wert in wässriger Lösung bei 25°C beträgt:

$$0 < pk_{CA} - pH_C \leqq 2,5$$

und der Bestandteil (B) eine Dissoziationskonstante $K_{CB}$ aufweisen, deren $pk_{CB}$-Wert in wässriger Lösung bei 25°C beträgt:

$$2 < pk_{CB} < pH_C.$$

und wobei die Bestandteile (A) und (B) das Färbevermögen des Färbemittels nicht inhibieren.

13. Zusammensetzung gemäß Anspruch 12, dadurch **gekennzeichnet** , daß die Verbindung der Formel (I) 5,6-Dihydroxyindol ist.

14. Zusammensetzung gemäß Anspruch 12, dadurch **gekennzeichnet** , daß die Verbindung der Formel (I) unter 2-Methyl-5,6-diyhdroxyindol, 3-Methyl-5,6-dihydroxyindol, 1-Methyl-5,6-dihydroxyindol, 2,3-Dimethyl-5,6-dihydroxyindol, 5-Methoxy-6-hydroxyindol ausgewählt ist.

15. Färbezusammensetzung für keratinische Fasern, insbesondere menschliches Haar, dadurch **gekennzeichnet** , daß sie in einem wässrigen Milieu mindestens 5,6-Dihydroxyindol und/oder seine Derivate der Formel (I)

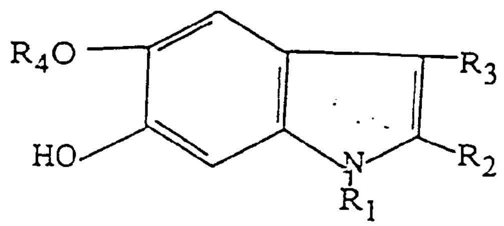

(I)

worin $R_1$, $R_2$, $R_3$, $R_4$, gleich oder verschieden, Wasserstoff oder eine $C_1$-$C_4$-Niedrigalkylgruppe bezeichnen, wobei $R_1$, $R_2$ und $R_3$ Wasserstoff bedeuten, wenn $R_4$ Alkyl bedeutet, sowie ein pH-Regulierungsmittel enthält, das es zuläßt, den pH auf einen $pH_C$-Wert von 5 bis 10 einzustellen, und das aus einem pH-Regulierungsmittel aus 2 Bestandteilen (A) und (B) zusammengesetzt ist, wobei der Bestandteil (A) aus Triethanolamin zusammengesetzt ist und der Bestandteil (B) eine Dissoziationskonstante $K_{CB}$ aufweist, so daß der $K_{CB}$-Wert in wässriger Lösung bei 25°C die Bedingung erfüllt:

$$2 < pk_{CB} < pH_C$$

und wobei der Bestandteil (A) und (B) das Färbevermögen des Färbemittels nicht inhibieren.

16. Zusammensetzung gemäß Anspruch 15, dadurch **gekennzeichnet** , daß die Verbindung der Formel (I) 5,6-Dihydroxyindol ist.

17. Zusammensetzung gemäß Anspruch 15, dadurch **gekennzeichnet** , daß die Verbindung der Formel (I) unter 2-Methyl-5,6-dihydroxyindol, 3-Methyl-5,6-dihydroxyindol, 1-Methyl-5,6-dihydroxyindol, 2,3-Dimethyl-5,6-dihydroxyindol, 5-Methoxy-6-hydroxyindol ausgewählt ist.

18. Zusammensetzung gemäß jedem der Ansprüche 15 bis 17, dadurch **gekennzeichnet** , daß der Bestandteil (B) Weinsäure mit einem $pk_{CB}$ von 3,2 ist.

19. Zusammensetzung gemäß jedem der Ansprüche 12 bis 18, dadurch **gekennzeichnet** , daß die Bestandteile (A) und (B) in einem Gewichtsverhältnis des Bestandteils (A) zum Bestandteil (B) von Größer als 1 und in molaren Verhältnissen des Bestandteils (A) zum Bestandteil (B) von größer oder gleich 2 und kleiner als 15 vorliegen.

20. Zusammensetzung gemäß jedem der Ansprüche 12 bis 19, dadurch **gekennzeichnet**, daß das 5,6-Dihydroxyindol und/oder seine Derivate der Formel (I) in Mengenverhältnissen von 0,01 bis 5 Gew.%, vorzugsweise von 0,2 bis 1 Gew.%, bezogen auf Gesantgewicht der Zusammensetzung, vorliegen.

21. Zusammentzung gemäß jedem der Ansprüche 12 bis 20, dadurch **gekennzeichnet**, daß die Zusammensetzung ein Lösungsmittel im Mengenverhältnis von 0,5 bis 50 Gew.%, vorzugsweise von 2 bis 20 Gew.%, enthält, ausgewählt unter $C_1$-$C_4$-Niedrigalkoholen, Ethylenglycol oder Propylenglycol, den Monomethyl-, Monoethyl- und Monobutylethern von Ethylenglycol, dem Acetat des Monoethylethers von Ethylenglycol, den Monomethylethern von Propylen- und Dipropylenglycol und Methyllactat.

22. Zuzammensetzung gemäß jedem der Ansprüche 12 bis 21, dadurch **gekennzeichnet**, daß die Zusammensetzung mit Hilfe von Verdickungsmitteln verdickt ist, die in Gewichtsverhältnissen von 0,1 bis 5%, vorzugsweise von 0,5 bis 3%, bezogen auf Gesantgewicht der Zusammensetzung, angewandt werden.

23. Zuzammensetzung gemäß jedem der Ansprüche 12 bis 22, dadurch **gekennzeichnet**, daß sie auch kationische, nicht-ionische, anionische, amphotere oberflächenaktive Mittel oder deren Mischungen in Mengenverhältnissen von 0,1 bis 50 Gew.%, besogen auf Gesamtgewicht der Zusammensetzung, enthält.

24. Verfahren zum Färben keratinischer Fasern, insbesondere von menschlichem Haar, dadurch **gekennzeichnet**, daß man die Zusammensetzung gemäß jedem der Ansprüche 12 bis 23 auf die genannten Fasern aufbringt und nach einer Verweilzeit von 1 bis 40 Minuten die genannten Fasern spült.

25. Verfahren zum Färben keratinischer Fasern, insbesondere von Haaren, dadurch **gekennzeichnet**, daß man eine fortschreitende Färbung der Haare durchführt, indem man mehrmals in aufeinanderfolgender Weise die Zusammensetzung gemäß jedem der Ansprüche 12 bis 23 aufbringt.

**Patentansprüche für folgende Vertragsstaaten : ES, GR**

1. Verfahren zur Bewahrung des Färbevermögens in wässrigem Milieu von 5,6-Dihydroxyindol und/oder seiner Derivate der Formel (I):

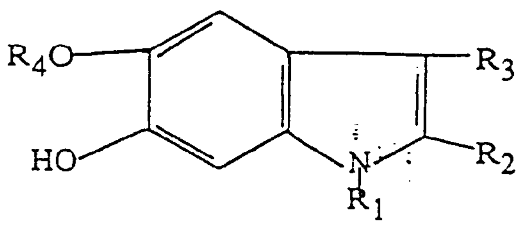

(I)

worin $R_1$, $R_2$, $R_3$, $R_4$, gleich oder verschieden, Wasserstoff oder eine $C_1$-$C_4$-Niedrigalkylgruppe bezeichnen, wobei $R_1$, $R_2$ und $R_3$ Wasserstoff bedeuten, wenn $R_4$ Alkyl bedeutet, dadurch **gekennzeichnet**, daß man den pH des wässrigen Milieus auf einen $pH_C$-Wert von 5 bis 10 einstellt, indem man ein pH-Regulierungsmittel aus 2 Bestandteilen (A) und (B) verwendet, wobei der Bestandteil (A) unter pH-Regulierungsmitteln mit einer Dissoziationskonstante $K_{CA}$, deren $pk_{CA}$-Wert in wässriger Lösung bei 25°C beträgt:

$$0 < pk_{CA} - pH_C \leqq 2,5$$

und der Bestandteil (B) unter pH-Regulierungsmitteln mit einer Dissoziationskonstante $K_{CB}$, deren $pk_{CB}$-Wert in wässriger Lösung bei 25°C beträgt:

$$2 < pk_{CB} < pH_C$$

ausgewählt sind.

2. Verfahren gemäß Anspruch 1, dadurch **gekennzeichnet**, daß die Verbindung der Formel (I) 5,6-Dihydroxyindol ist.

3. Verfahren gemäß Anspruch 1, dadurch **gekennzeichnet**, daß die Verbindung der Formel (I) unter 2-Methyl-5,6-dihydroxyindol, 3-Methyl-5,6-dihydroxyindol, 1-Methyl-5,6-dihydroxyindol, 2,3-Dimethyl-5,6-dihydroxyindol, 5-Methoxy-6-hydroxyindol ausgewählt ist.

4. Verfahren zur Bewahrung des Färbevermögens in wässrigem Milieu von 5,6-Dihydroxyindol und/oder seiner Derivate der Formel (I):

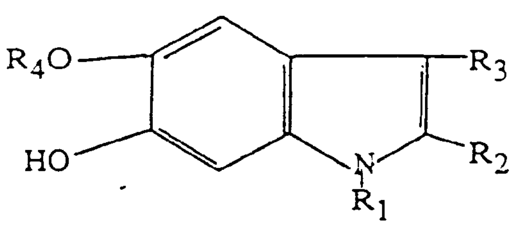

(I)

worin $R_1$, $R_2$, $R_3$, $R_4$, gleich oder verschieden, Wasserstoff oder eine $C_1$-$C_4$-Niedrigalkylgruppe bezeichnen, wobei $R_1$, $R_2$ und $R_3$ Wasserstoff bedeuten, wenn $R_4$ Alkyl bedeutet, dadurch **gekennzeichnet**, daß man den pH des wässrigen Milieus auf einen $pH_C$-Wert von 5 bis 10 einstellt, indem man ein pH-Regulierungsmittel aus 2 Bestandteilen (A) und (B) verwendet, wobei der Bestandteil (A) Triethanolamin ist und der Bestandteil (B) unter pH-Regulierungsmitteln mit einer Dissoziationskonstante $K_{CB}$ ausgewählt ist, deren $pk_{CB}$-Wert in wässriger Lösung bei 25°C:

$$2 < pk_{CB} < pH_C$$

beträgt.

5. Verfahren gemäß Anspruch 4, dadurch **gekennzeichnet**, daß der Bestandteil (B) aus Weinsäure mit einem $pk_{CB}$-Wert von 3,2 zusammengesetzt ist.

6. Verfahren gemäß Anspruch 4 oder 5, dadurch **gekennzeichnet**, daß die Verbindung der Formel (I) 5,6-Dihydroxyindol ist

7. Verfahren gemäß Anspruch 4 oder 5, dadurch **gekennzeichnet**, daß die Verbindung der Formel (I) unter 2-Methyl-5,6-dihydroxyindol, 3-Methyl-5,6-dihydroxyindol, 1-Methyl-5,6-dihydroxyindol, 2,3-Dimethyl-5,6-dihydroxyindol, 5-Methoxy-6-hydroxyindol ausgewählt ist.

8. Verfahren gemäß jedem der Ansprüche 1 bis 3, dadurch **gekennzeichnet**, daß der Bestandteil (A) aus saurem Dikaliumphosphat mit einem $pk_{CA}$-Wert von 7,2 und der Bestandteil (B) aus Kaliumdihydrogenphosphat mit einem $pk_{CB}$-Wert von 2,1 zusammengesetzt sind.

9. Verfahren gemäß jedem der Ansprüche 1 bis 8, dadurch **gekennzeichnet**, daß das Gewichtsverhältnis des Bestandteils (A) zum Bestandteil (B) größer als 1 und das molare Verhältnis des Bestandteils (A) zum Bestandteil (B) größer oder gleich 2 und kleiner als 15 sind.

10. Verfahren gemäß jedem der Ansprüche 1 bis 9, dadurch **gekennzeichnet**, daß das pH-Regulierungsmittel aus den zwei Bestandteilen (A) und (B) in Mengenverhältnissen von $5 \times 10^{-3}$ bis $50 \times 10^{-3}$ Mol pro 100 g Färbezusammensetzung vorliegt.

11. Verfahren gemäß jedem der Ansprüche 1 bis 10, dadurch **gekennzeichnet**, daß der pH des Milieus einen $pH_C$-Wert von 7 bis 9 aufweist.

12. Färbezusammensetzung für keratinische Fasern, insbesondere menschliches Haar, dadurch **gekennzeichnet**, daß sie in einem wässrigen Milieu mindestens 5,6-Dihydroxyindol und/oder seine Derivate der Formel (I):

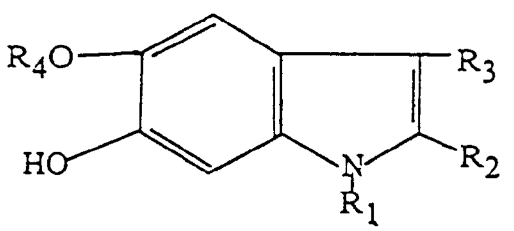

(I)

worin $R_1$, $R_2$, $R_3$, $R_4$, gleich oder verschieden, Wasserstoff oder eine $C_1$-$C_4$-Niedrigalkylgruppe bezeichnen, wobei $R_1$, $R_2$ und $R_3$ Wasserstoff bedeuten, wenn $R_4$ Alkyl bedeutet, sowie ein pH-Regulierungsmittel enthält, welches es zuläßt, den pH auf einen $pH_C$-Wert von 5 bis 10 einzustellen, und das aus einem pH-Regulierungsmittel aus 2 Bestandteilen (A) und (B) zusammengesetzt ist, wobei der Bestandteil (A) eine Dissoziationskonstante $K_{CA}$, deren $pk_{CA}$-Wert in wässriger Lösung bei 25°C beträgt:

$$0 < pk_{CA} - pH_C \leqq 2,5$$

und der Bestandteil (B) eine Dissoziationskonstante $K_{CB}$ aufweisen, deren $pk_{CB}$-Wert in wässriger Lösung bei 25°C beträgt:

$$2 < pk_{CB} < pH_C.$$

und wobei die Bestandteile (A) und (B) das Färbevermögen des Färbemittels nicht inhibieren.

13. Zusammensetzung gemäß Anspruch 12, dadurch **gekennzeichnet**, daß die Verbindung der Formel (I) 5,6-Dihydroxyindol ist.

14. Zusammensetzung gemäß Anspruch 12, dadurch **gekennzeichnet**, daß die Verbindung der Formel (I) unter 2-Methyl-5,6-diyhdroxyindol, 3-Methyl-5,6-dihydroxyindol, 1-Methyl-5,6-dihydroxyindol, 2,3-Dimethyl-5,6-dihydroxyindol, 5-Methoxy-6-hydroxyindol ausgewählt ist.

15. Färbezusammensetzung für keratinische Fasern, insbesondere menschliches Haar, dadurch **gekennzeichnet**, daß sie in einem wässrigen Milieu mindestens 5,6-Dihydroxyindol und/oder seine Derivate der Formel (I)

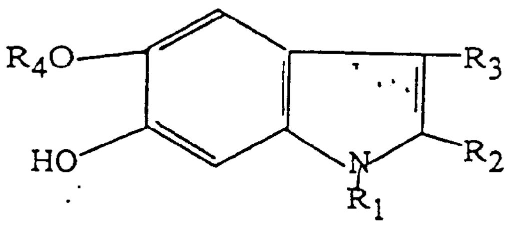

(I)

worin $R_1$, $R_2$, $R_3$, $R_4$ ,gleich oder verschieden, Wasserstoff oder eine $C_1$-$C_4$-Niedrigalkylgruppe bezeichnen, wobei $R_1$, $R_2$ und $R_3$ Wasserstoff bedeuten, wenn $R_4$ Alkyl bedeutet, sowie ein pH-Regulierungsmittel enthält,

das es zuläßt, den pH auf einen $pH_C$-Wert von 5 bis 10 einzustellen, und das aus einem pH-Regulierungsmittel aus 2 Bestandteilen (A) und (B) zusammengesetzt ist, wobei der Bestandteil (A) aus Triethanolamin zusammengesetzt ist und der Bestandteil (B) eine Dissoziationskonstante $K_{CB}$ aufweist, so daß der $K_{CB}$-Wert in wässriger Lösung bei 25°C die Bedingung erfüllt:

$$2 < pk_{CB} < pH_C$$

und wobei der Bestandteil (A) und (B) das Färbevermögen des Färbemittels nicht inhibieren

16. Zusammensetzung gemäß Anspruch 15, dadurch **gekennzeichnet** , daß die Verbindung der Formel (I) 5,6-Dihydroxyindol ist.

17. Zusammensetzung gemäß Anspruch 15, dadurch **gekennzeichnet** , daß die Verbindung der Formel (I) unter 2-Methyl-5,6-dihydroxyindol, 3-Methyl-5,6-dihydroxyindol, 1-Methyl-5,6-dihydroxyindol, 2,3-Dimethyl-5,6-dihydroxyindol, 5-Methoxy-6-hydroxyindol ausgewählt ist.

18. Zusammensetzung gemäß jedem der Ansprüche 15 bis 17, dadurch **gekennzeichnet** , daß der Bestandteil (B) Weinsäure mit einem $pk_{CB}$ von 3,2 ist.

19. Zusammensetzung gemäß jedem der Ansprüche 12 bis 18, dadurch **gekennzeichnet** , daß die Bestandteile (A) und (B) in einem Gewichtsverhältnis des Bestandteils (A) zum Bestandteil (B) von größer als 1 und in molaren Verhältnissen des Bestandteils (A) zum Bestandteil (B) von größer oder gleich 2 und kleiner als 15 vorliegen.

20. Zusammensetzung gemäß jedem der Ansprüche 12 bis 19, dadurch **gekennzeichnet** , daß das 5,6-Dihydroxyindol und/oder seine Derivate der Formel (I) in Mengenverhältnissen von 0,01 bis 5 Gew.%, vorzugsweise von 0,2 bis 1 Gew.%, bezogen auf Gesamtgewicht der Zusammensetzung, vorliegen.

21. Zusammensetzung gemäß jedem der Ansprüche 12 bis 20, dadurch **gekennzeichnet** , daß die Zusammensetzung ein Lösungsmittel im Mengenverhältnis von 0,5 bis 50 Gew.%, vorzugsweise von 2 bis 20 Gew.%, enthält, ausgewählt unter $C_1$-$C_4$-Niedrigalkoholen, Ethylenglycol oder Propylenglycol, den Monomethyl-, Monoethyl- und Monobutylethern von Ethylenglycol, dem Acetat des Monoethylethers von Ethylenglycol, den Monomethylethern von Propylen- und Dipropylenglycol und Methyllactat.

22. Zusammensetzung gemäß jedem der Ansprüche 12 bis 21, dadurch **gekennzeichnet** , daß die Zusammensetzung mit Hilfe von Verdickungsmitteln verdickt ist, die in Gewichtsverhältnissen von 0,1 bis 5%, vorzugsweise von 0,5 bis 3%, bezogen auf Gesamtgewicht der Zusammensetzung, angewandt werden.

23. Zusammensetzung gemäß jedem der Ansprüche 12 bis 22, dadurch **gekennzeichnet** , daß sie auch kationische, nicht-ionische, anionische, amphotere oberflächenaktive Mittel oder deren Mischungen in Mengenverhältnissen von 0,1 bis 50 Gew.%, bezogen auf Gesamtgewicht der Zusammensetzung, enthält.

24. Verfahren zum Färben keratinischer Fasern, insbesondere von menschlichem Haar, dadurch **gekennzeichnet** , daß man die Zusammensetzung gemäß jedem der Ansprüche 12 bis 23 auf die genannten Fasern aufbringt und nach einer Verweilzeit von 1 bis 40 Minuten die genannten Fasern spült.

25. Verfahren zum Färben keratinischer Fasern, insbesondere von Haaren, dadurch **gekennzeichnet** , daß man eine fortschreitende Färbung der Haare durchführt, indem man mehrmals in aufeinanderfolgender Weise die Zusammensetzung gemäß jedem der Ansprüche 12 bis 23 aufbringt.

26. Verfahren zur Herstellung einer Färbezusammensetzung für keratinische Fasern, insbesondere für menschliches Haar, dadurch **gekennzeichnet** , daß man in ein wässriges Milieu mindestens 5,6-Dihydroxyindol und/oder seine Derivate der Formel (I):

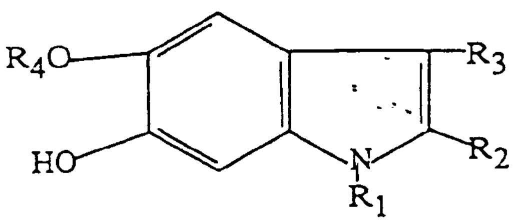

(I)

worin $R_1$, $R_2$, $R_3$, $R_4$, gleich oder verschieden, Wasserstoff oder eine $C_1$-$C_4$-Niedrigalkylgruppe bezeichnen, wobei $R_1$, $R_2$ und $R_3$ Wasserstoff bedeuten, wenn $R_4$ Alkyl bedeutet, in Mengenverhältnissen von 0,01 bis 5 Gew.%, bezogen auf Gesamtgewicht der Zusammensetzung, sowie ein pH-Regulierungsmittel einbringt, das es ermöglicht, den pH auf einen $pH_C$-Wert von 5 bis 10 einzustellen, und welches aus einem pH-Regulierungsmittel aus zwei Bestandteilen (A) und (B) zusammengesetzt ist, wobei der Bestandteil (A) eine Dissoziationskonstante $K_{CA}$ aufweist, so daß der $pk_{CA}$-Wert in wässriger Lösung bei 25°C die Bedingung erfüllt:

$$0 < pk_{CA} - pH_C \leqq 2{,}5$$

und der Bestandteil (B) eine Dissoziationskonstante $K_{CB}$ aufweist, so daß der $pk_{CB}$-Wert in wässriger Lösung

bei 25°C die Bedingung erfüllt:

$$2 < pk_{CB} < pH_C$$

und wobei die Bestandteile (A) und (B) das Färbevermögen des Färbemittels nicht inhibieren.

27. Verfahren zur Herstellung einer Färbezusammensetzung für keratinische Fasern, insbesondere für menschliches Haar, dadurch **gekennzeichnet** , daß man in ein wässriges Milieu mindestens 5,6-Dihydroxyindol und/oder seine Derivate der Formel (I):

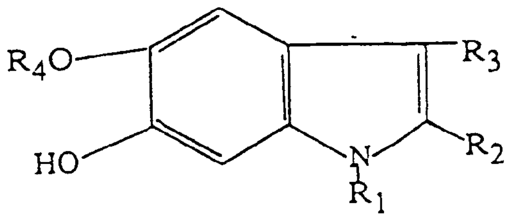

(I)

worin $R_1$, $R_2$, $R_3$, $R_4$, gleich oder verschieden, Wasserstoff oder eine $C_1$-$C_4$-Niedrigalkylgruppe bezeichnen, wobei $R_1$, $R_2$ und $R_3$ Wasserstoff bedeuten, wenn $R_4$ Alkyl bedeutet, in Mengenverhältnissen von 0,01 bis 5 Gew.%, bezogen auf Gesamtgewicht der Zusammensetzung, sowie ein pH-Regulierungsmittel einbringt, das es ermöglicht, den pH auf einen $pH_C$-Wert von 5 bis 10 einzustellen, und welches aus einem pH-Regulierungsmittel aus 2 Bestandteilen (A) und (B) zusammengesetzt ist, wobei der Bestandteil (A) Triethanolamin ist und der Bestandteil (B) eine Dissoziationskonstante $K_{CB}$ aufweist, so daß der $pk_{CB}$-Wert in wässriger Lösung bei 25°C die Bedingung erfüllt:

$$2 < pk_{CB} < pH_C$$

und wobei die Bestandteile (A) und (B) das Färbevermögen des Färbemittels nicht inhibieren.

## Claims

### Claims for the following Contracting States : DE, GB, FR, IT, NL, SE, CH, BE, AT

1. Process for preserving the dyeing power of 5,6-dihydroxyindole and/or derivatives thereof, of the formula (I) :

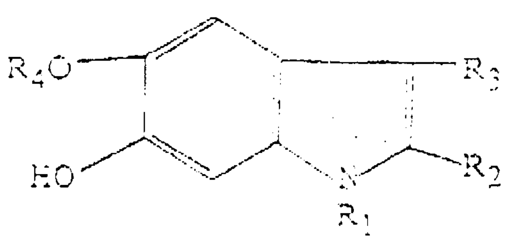

(I)

in which $R_1$, $R_2$, $R_3$ and $R_4$, which are identical or different, denote hydrogen or lower $C_1$-$C_4$ alkyl, $R_1$, $R_2$ and $R_3$ denoting hydrogen when $R_4$ denotes alkyl, in an aqueous medium, characterised in that the pH of the aqueous medium is adjusted to a $pH_C$ value of between 5 and 10 using a pH-regulating agent containing two components (A) and (B), the component (A) being chosen from pH-regulating agents having a dissociation constant $K_{CA}$, in aqueous solution at 25°C, such that the $pk_{CA}$ is:

$$O < pk_{CA} - pH_C \leqq 2.5$$

and the component (B) being chosen from pH-regulating agents having a dissociation constant $K_{CB}$, in aqueous solution at 25°C, such that the $pk_{CB}$ is:

$$2 < pk_{CB} < pH_C.$$

2. Process according to Claim 1, characterised in that the compound of formula (I) is 5,6-dihydroxyindole.

3. Process according to Claim 1, characterised in that the compound of formula (I) is chosen from 2-methyl-5,6-dihydroxyindole, 3-methyl-5,6-dihydroxyindole, 1-methyl-5,6-dihydroxyindole, 2,3-dimethyl-5,6-dihydroxyindole and 5-methoxy-6-hydroxyindole.

4. Process for preserving the dyeing power of 5,6-dihydroxyindole and/or derivatives thereof, of the formula (I):

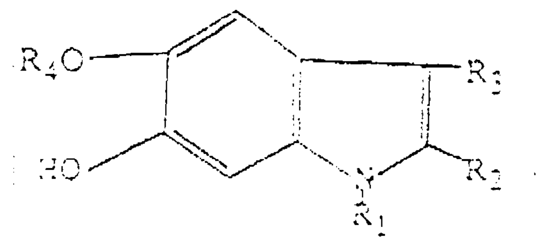

(I)

in which $R_1$, $R_2$, $R_3$ and $R_4$, which are identical or different, denote hydrogen or lower $C_1$-$C_4$ alkyl, $R_1$, $R_2$ and $R_3$ denoting hydrogen when $R_4$ denotes alkyl, in an aqueous medium, characterised in that the pH of the aqueous medium is adjusted to a $pH_C$ value of between 5 and 10 using a pH-regulating agent containing two components (A) and (B), the component (A) being triethanolamine and the component (B) being chosen from pH-regulating agents having a dissociation constant $K_{CB}$, in aqueous solution at 25°C, such that the $pk_{CB}$ is:

$$2 < pk_{CB} < pH_C.$$

5. Process according to Claim 4, characterised in that the component (B) consists of tartaric acid having a $pk_{CB}$ value of 3.2.

6. Process according to Claim 4 or 5, characterised in that the compound of formula (I) is 5,6-dihydroxyindole.

7. Process according to Claim 4 or 5, characterised in that the compound of formula (I) is chosen from 2-methyl-5,6-dihydroxyindole, 3-methyl-5,6-dihydroxyindole, 1-methyl-5,6-dihydroxyindole, 2,3-dimethyl-5,6-dihydroxyindole and 5-methoxy-6-hydroxyindole.

8. Process according to any one of Claims 1 to 3, characterised in that the component (A) consists of dipotassium acid phosphate having a $pk_{CA}$ value of 7.2 and in that the component (B) consists of potassium diacid phosphate having a $pk_{CB}$ value of 2.1.

9. Process according to any one of Claims 1 to 8, characterised in that the weight ratio of the component (A) to the component (B) is greater than 1 and in that the mole ratio of the component (A) to the component (B) is not less than 2 and less than 15.

10. Process according to any one of Claims 1 to 9, characterised in that the pH-regulating agent containing two components (A) and (B) is present in proportions of between $5.10^{-3}$ and $50.10^{-3}$ moles per 100 g of dyeing composition.

11. Process according to any of Claims 1 to 10, characterised in that the pH of the medium has a $pH_C$ value of between 7 and 9.

12. Dyeing composition for keratinous fibres, in particular for human hair, characterised in that it contains, in an aqueous medium, at least 5,6-dihydroxyindole and/or derivatives thereof, of formula (I) :

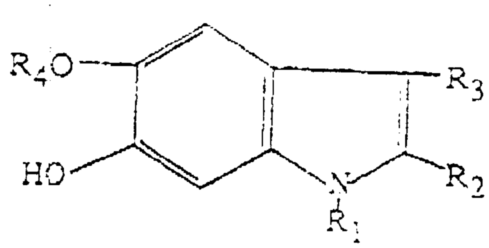

(I)

in which $R_1$, $R_2$, $R_3$ and $R_4$, which are identical or different, denote hydrogen or lower $C_1$-$C_4$ alkyl, $R_1$, $R_2$ and $R_3$ denoting hydrogen when $R_4$ denotes alkyl, and a pH-regulating agent enabling the pH to be adjusted to a $pH_C$ value of between 5 and 10, which consists of a pH-regulating agent containing two components (A) and (B), the component (A) having a dissociation constant $K_{CA}$, in aqueous solution at 25°C, such that $pk_{CA}$ is:

$$O < pk_{CA} - pH_C \leqq 2.5$$

and the component (B) having a dissociation constant $K_{CB}$, in aqueous solution at 25°C, such that $pk_{CB}$ is:

$$2 < pk_{CB} < pH_C,$$

the components (A) and (B) not inhibiting the dyeing power of the colourant.

13. Composition according to Claim 12, characterised in that the compound of formula (I) is 5,6-dihydroxyindole.

14. Composition according to Claim 12, characterised in that the compound of formula (I) is chosen from 2-methyl-5,6-dihydroxyindole, 3-methyl-5,6-dihydroxyindole, 1-methyl-5,6-dihydroxyindole, 2,3-dimethyl-5,6-dihydroxyindole and 5-methoxy-6-hydroxyindole.

15. Dyeing composition for keratinous fibres, in particular for human hair, characterised in that it contains, in an aqueous medium, at least 5,6-dihydroxyindole and/or derivatives thereof, of formula (I):

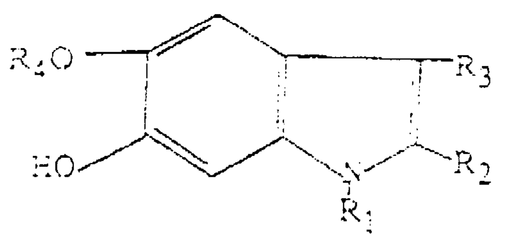

(I)

in which $R_1$, $R_2$, $R_3$ and $R_4$, which are identical or different, denote hydrogen or lower $C_1$-$C_4$ alkyl, $R_1$, $R_2$ and $R_3$ denoting hydrogen when $R_4$ denotes alkyl, and a pH-regulating agent enabling the pH to be adjusted to a $pH_C$ value of between 5 and 10, which consists of a pH-regulating agent containing two components (A) and (B), the component (A) consisting of triethanolamine and the component (B) having a dissociation constant $K_{CB}$, in aqueous solution at 25°C, such that $pk_{CB}$ is:

$$2 < pk_{CH} < pH_C$$

the components (A) and (B) not inhibiting the dyeing power of the colourant.

16. Composition according to Claim 15, characterised in that the compound of formula (I) is 5,6-dihydroxyindole.

17. Composition according to Claim 15, characterised in that the compound of formula (I) is chosen from 2-methyl-5,6-dihydroxyindole, 3-methyl-5,6-dihydroxyindole, 1-methyl-5,6-dihydroxyindole, 2,3-dimethyl-5,6-dihydroxyindole and 5-methoxy-6-hydroxyindole.

18. Composition according to any one of Claims 15 to 17, characterised in that the component (B) is tartaric acid having a $pk_{CB}$ value of 3.2.

19. Composition according to any one of Claims 12 to 18, characterised in that the components (A) and (B) are present in proportions by weight such that the ratio of the component (A) to the component (B) is greater than 1, and in molar proportions such that the ratio of the component (A) to the component (B) is not less than 2 and less than 15.

20. Composition according to any one of Claims 12 to 19, characterised in that the 5,6-dihydroxyindole and/or derivatives thereof, of formula (I), is (are) present in proportions of between 0.01 and 5 % by weight, and preferably between 0.2 and 1 % by weight, relative to the total weight of the composition.

21. Composition accordingj to any one of Claims 12 to 20, characterised in that the composition contains a solvent in proportions of between 0.5 and 50 % by weight, preferably 2 to 20 % by weight, chosen from lower $C_1$-$C_4$ alcohols, ethylene glycol or propylene glycol, ethylene glycol monomethyl, monoethyl and monobytyl ethers, ethylene glycol monoethyl ether acetate, propylene glycol and dipropylene glycol monomethyl ethers and methyl lactate.

22. Composition according to any one of Claims 12 to 21, characterised in that the composition is thickened by means of thickening agents used in proportions by weight of between 0.1 and 5 %, and preferably between 0.5 and 3 %, relative to the total weight of the composition.

23. Composition according to any one of Claims 12 to 22, characterised in that it also contains cationic, nonionic, anionic or amphoteric surface-active agents or mixtures thereof, in proportions of between 0.1 and 50 % by weight relative to the total weight of the composition.

24. Process for dyeing keratinous fibres, in particular human hair, characterised in that the composition as defined in any one of Claims 12 to 23 is applied to the said fibres and in that after an exposure time of between 1 minute and 40 minutes the said fibres are rinsed.

25. Process for dyeing keratinous fibres, in particular hair, characterised in that the hair is dyed gradually by applying several times, and successively, the composition as defined in any one of Claims 12 to 23.

**Claims for the following Contracting States : ES, GR**

1. Process for preserving the dyeing power of 5,6-dihydroxyindole and/or derivatives thereof, of the formula (I) :

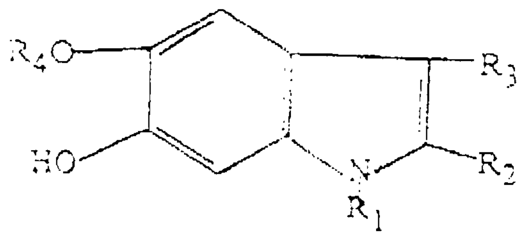

(I)

in which $R_1$, $R_2$, $R_3$ and $R_4$, which are identical or different, denote hydrogen or lower $C_1$-$C_4$ alkyl, $R_1$, $R_2$ and $R_3$ denoting hydrogen when $R_4$ denotes alkyl, in an aqueous medium, characterised in that the pH of the aqueous medium is adjusted to a $pH_C$ value of between 5 and 10 using a pH-regulating agent containing two components (A) and (B), the component (A) being chosen from pH-regulating agents having a dissociation constant $K_{CA}$, in aqueous solution at 25°C, such that the $pk_{CA}$ is:

$$O < pk_{CA} - pH_C \leqq 2.5$$

and the component (B) being chosen from pH-regulating agents having a dissociation constant $K_{CB}$, in aqueous solution at 25°C, such that $pk_{CB}$ is:

$$2 < pk_{CB} < pH_C.$$

2. Process according to Claim 1, characterised in that the compound of formula (I) is 5,6-dihydroxyindole.

3. Process according to Claim 1, characterised in that the compound of formula (I) is chosen from 2-methyl-5,6-dihydroxyindole, 3-methyl-5,6-dihydroxyindole, 1-methyl-5,6-dihydroxyindole, 2,3-dimethyl-5,6-dihydroxyindole and 5-methoxy-6-hydroxyindole.

4. Process for preserving the dyeing power of 5,6-dihydroxyindole and/or derivatives thereof, of the formula (I) :

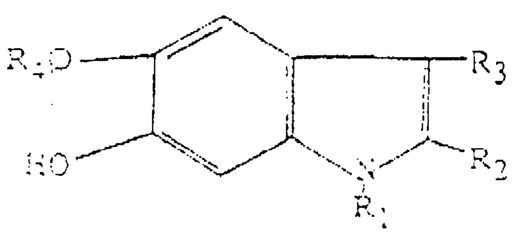

(I)

in which $R_1$, $R_2$, $R_3$ and $R_4$, which are identical or different, denote hydrogen or lower $C_1$-$C_4$ alkyl, $R_1$, $R_2$ and $R_3$ denoting hydrogen when $R_4$ denotes alkyl, in an aqueous medium, characterised in that the pH of the aqueous medium is adjusted to a $pH_C$ value of between 5 and 10 using a pH-regulating agent containing two components (A) and (B), the component (A) being triethanolamine and the component (B) being chosen from pH-regulating agents having a dissociation constant $K_{CB}$, in aqueous solution at 25°C, such that the $pk_{CB}$ is:

$$2 < pk_{CB} < pH_C.$$

5. Process according to Claim 4, characterised in that the component (B) consists of tartaric acid having a $pk_{CB}$ value of 3.2.

6. Process according to Claim 4 or 5, characterised in that the compound of formula (I) is 5,6-dihydroxyindole.

7. Process according to Claim 4 or 5, characterised in that the compound of formula (I) is chosen from 2-methyl-5,6-dihyddroxyindole, 3-methyl-5,6-dihydroxyindole, 1-methyl-5,6-dihydroxyindole, 2,3-dimethyl-5,6-dihydroxyindole and 5-methoxy-6-hydroxyindole.

8. Process according to any one of Claims 1 to 3, characterised in that the component (A) consists of dipotassium acid phosphate having a $pk_{CA}$ value of 7.2 and in that the component (B) consists of potassium diacid phosphate having a $pk_{CB}$ value of 2.1.

9. Process according to any one of Claims 1 to 8, characterised in that the weight ratio of the component (A) to the component (B) is greater than 1 and in that the mole ratio of the component (A) to the component (B) is not less than 2 and less than 15.

10. Process according to any one of Claim 1 to 9, characterised in that the pH-regulating agent containing two components (A) and (B) is present in proportions of between $5 \times 10^{-3}$ and $50 \times 10^{-3}$ moles per 100 g of dyeing composition.

11. Process according to any one of Claim 1 to 10, characterised in that the pH of the medium has a $pH_C$

value of between 7 and 9.

12. Dyeing composition for keratinous fibres, in particular for human hair, characterised in that it contains, in an aqueous medium, at least 5,6-dihydroxyindole and/or derivatives thereof, of formula (I):

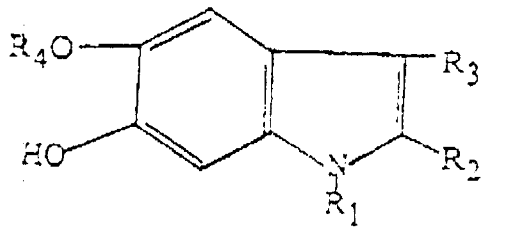

(I)

in which $R_1$, $R_2$, $R_3$ and $R_4$, which are identical or different, denote hydrogen or lower $C_1$-$C_4$ alkyl, $R_1$, $R_2$ and $R_3$ denoting hydrogen when $R_4$ denotes alkyl, and a pH-regulating agent enabling the pH to be adjusted to a $pH_C$ value of between 5 and 10, which consists of a pH-regulating agent containing two components (A) and (B), the component (A) having a dissociation constant $K_{CA}$, in aqueous solution at 25°C, such that $pk_{CA}$ is:

$$O < pk_{CA} - pH_C \leqq 2.5$$

and the component (B) having a dissociation constant $K_{CB}$, in aqueous solution at 25°C, such that $pk_{CB}$ is:

$$2 < pk_{CB} < pH_C,$$

the components (A) and (B) not inhibiting the dyeing power of the colourant.

13. Composition according to Claim 12, characterised in that the compound of formula (I) is 5,6-dihydroxyindole.

14. Composition according to Claim 12, characterised in that the compound of formula (I) is chosen from 2-methyl-5,6-dihydroxyindole, 3-methyl-5,6-dihydroxyindole, 1-methyl-5,6-dihydroxyindole, 2,3-dimethyl-5,6-dihydroxyindole and 5-methoxy-6-hydroxyindole.

15. Dyeing composition for keratinous fibres, in particular for human hair, characterised in that it contains, in an aqueous medium, at least 5,6-dihydroxyindole and/or derivatives thereof, of formula (I):

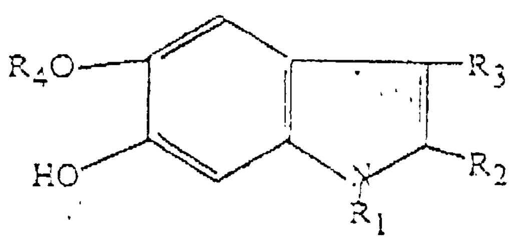

(I)

in which $R_1$, $R_2$, $R_3$ and $R_4$, which are identical or different, denote hydrogen or lower $C_1$-$C_4$ alkyl, $R_1$, $R_2$ and $R_3$ denoting hydrogen when $R_4$ denotes alkyl, and a pH-regulating agent enabling the pH to be adjusted to a $pH_C$ value of between 5 and 10, which consists of a pH-regulating agent containing two components (A) and (B), the component (A) consisting of triethanolamine and the component (B) having a dissociation constant $K_{CB}$, in aqueous solution at 25°C, such that $pk_{CB}$ is:

$$2 < pk_{CB} < pH_C$$

the components (A) and (B) not inhibiting the dyeing power of the colourant.

16. Composition according to Claim 15, characterised in that the compound of formula (I) is 5,6-dihydroxyindole.

17. Composition according to Claim 15, characterised in that the compound of formula (I) is chosen from 2-methyl-5,6-dihydroxyindole, 3-methyl-5,6-dihydroxyindole, 1-methyl-5,6-dihydroxyindole, 2,3-dimethyl-5,6-dihydroxyindole and 5-methoxy-6-hydroxyindole.

18. Composition according to any one of Claims 15 to 17, characterised in that the component (B) is tartaric acid having a $pk_{CB}$ value of 3.2.

19. Composition according to any one of Claims 12 to 18, characterised in that the components (A) and (B) are present in proportions by weight such that the ratio of the component (A) to the component (B) is greater than 1, and in molar proportions such that the ratio of the component (A) to the component (B) is not less than 2 and less than 15.

20. Composition according to any one of Claims 12 to 19, characterised in that the 5,6-dihydroxyindole and/or derivatives thereof, of formula (I), is (are) present in proportions of between 0.01 and 5 % by weight,

and preferably between 0.2 and 1 % by weight, relative to the tatal weightd of the composition.

21. Composition according to any one of Claims 12 to 20, characterised in that the composition contains a solvent in proportions of between 0.5 and 50 % by weight, preferably 2 to 20 % by weight, chosen from lower $C_1$-$C_4$ alcohols, ethylene glycol or propylene glycol, ethylene glycol monomethyl, monoethyl and monobutyl ethers, ethylene glycol monoethyl ether acetate, propylene glycol and dipropylene glycol monomethyl ethers and methyl lactate.

22. Composition according to any one of Claims 12 to 21, characterised in that the composition is thickened by means of thickening agents used in proportions by weight of between 0.1 and 5 %, and preferably between 0.5 and 3 %, relative to the total weight of the composition.

23. Composition according to any one of Claims 12 to 22, characterised in that it also contains cationic, nonionic, anionic or amphoteric surface-active agents or mixtures thereof, in proportions of between 0.1 and 50 % by weight relative to the total weight of the composition.

24. Process for dyeing keratinous fibres, in particular human air, characterised in that the composition as defined in any one of Claims 12 to 23 is applied to the said fibres and in that after an exposure time of between 1 minute and 40 minutes the said fibres are rinsed.

25. Process for dyeing keratinous fibres, in particular hair, characterised in that the hair is dyed gradually by applying several times, and successively, the composition as defined in any one of Claims 12 to 23.

26. Process for preparing a dyeing composition for keratinous fibres, in particular for human hair, characterised in that the following are introduced into an aqueous medium: at least 5,6-dihydroxyindole and/or derivatives thereof, of formula (I):

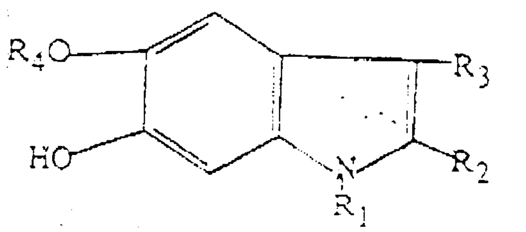

(I)

in which $R_1$, $R_2$, $R_3$ and $R_4$, which are identical or different, denote hydrogen or lower $C_1$-$C_4$ alkyl, $R_1$, $R_2$ and $R_3$ denoting hydrogen when $R_4$ denotes alkyl, in proportions of between 0.01 and 5 % by weight relative to the total weight of the composition, and a pH-regulating agent enabling the pH to be adjusted to a $pH_C$ value of between 5 and 10, which consists of a pH-regulating agent containing two components (A) and (B), the component (A) having a dissociation constant $K_{CA}$, in aqueous solution at 25°C, such that $pk_{CA}$ is:

$$O < pk_{CA} - pH_C \leqq 2.5$$

and the component (B) having a dissociation constant $K_{CB}$, in aqueous solution at 25°C, such that $pk_{CB}$ is:

$$2 < pk_{CB} < pH_C,$$

the components (A) and (B) not inhibiting the dyeing power of the colourant.

27. Process for preparing a dyeing composition for keratinous fibres, in particular for human hair, characterised in that the following are introduced into an aqueous medium; at least 5,6-dihydroxyindole and/or derivatives thereof, of formula (I):

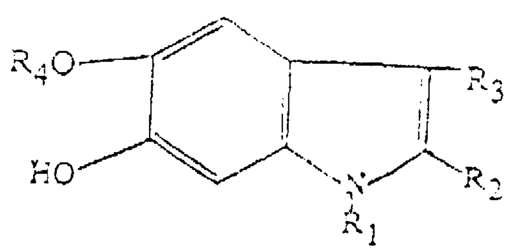

(I)

in which $R_1$, $R_2$, $R_3$ and $R_4$ , which are identical or different, denote hydrogen or lower $C_1$-$C_4$ alkyl, $R_1$, $R_2$ and $R_3$ denoting hydrogen when $R_4$ denotes alkyl, in proportions of between 0.01 and 5 % by weight relative to the total weight of the composition, and a pH-regulating agent enabling the pH to be adjusted to a $pH_C$ value of between 5 and 10, which consists of a pH-regulating agent containing two components (A) and (B), the component (A) being triethanolamine and the component (B) having a dissociation constant $K_{CB}$, in aqueous solution at 25°C, such that $pk_{CB}$ is:

$$2 < pk_{CB} < pH_C$$

the components (A) and (B) not inhibiting the dyeing power of the colourant.